# EUROPEAN PATENT APPLICATION

(11) **EP 1 715 048 A1**
(43) Date of publication of application: **25.10.2006**
(21) Application number: 05703936.4
(22) Date of filing: 20.01.2005
(51) Int. Cl.: C12N 15/45, C12N 15/64, C12N 7/00

(54) **PROCESS FOR PRODUCING VIRUS VECTOR**

(30) Priority: 22.01.2004 JP 2004014654
(71) Applicant: Dnavec Research Inc., Tsukuba-shi, Ibaraki 305-0856 (JP)
(72) Inventor: INOUE, Makoto, Tsukuba-shi, Ibaraki 3050856 (JP); BAN, Hiroshi, Tsukuba-shi, Ibaraki 3050856 (JP); IIDA, Akihiro, Tsukuba-shi, Ibaraki 3050856 (JP); HASEGAWA, Mamoru, Tsukuba-shi, Ibaraki 3050856 (JP)
(74) Representative: Vossius & Partner
(86) International application number: PCT/JP2005/000708
(87) International publication number: WO 2005/071085

(57) **Abstract**

The present invention provides methods for producing viruses, whose propagation depends on the cleavage of viral protein by a protease, in a manner independent of the protease. The methods of the present invention for producing viruses comprise producing viruses in the presence of a modified viral protein in which the protease cleavage sequence is changed to a cleavage sequence for an alternative protease. Viral vectors can be more efficiently produced by replacing the protease cleavage sequence with a cleavage sequence for a protease expressed endogenously in virus-producing cells. The methods of the present invention enable the production of high titer viruses using a wide variety of cells.

SEQUENCE LISTING

<110>  DNAVEC RESEARCH INC.

<120>  Process for producing virus vector

<130>  D3-A0401P

<140>
<141>

<150>  JP  2004-014654
<151>  2004-01-22

<160>  54

<170>  PatentIn version 3.1

<210>  1
<211>  6
<212>  PRT
<213>  Artificial

<220>
<223>  an example of protease cleavage sequence

<400>  1
Pro Leu Gly Met Thr Ser
1               5

<210>  2
<211>  6
<212>  PRT
<213>  Artificial

<220>
<223>  an example of protease cleavage sequence

<400>  2
Pro Gln Gly Met Thr Ser
1               5

<210>  3
<211>  7
<212>  PRT
<213>  Artificial

<220>
<223>  an example of protease cleavage sequence

<400>  3
Pro Leu Gly Leu Trp Ala Arg
1               5

<210>  4
<211>  8
<212>  PRT
<213>  Artificial

<220>
<223>  an example of protease cleavage sequence

<400>  4
Gly Pro Leu Gly Met Arg Gly Leu
1               5

<210>  5
<211>  7

<212>  PRT
<213>  Artificial

<220>
<223>  an example of protease cleavage sequence

<400>  5
Pro Gln Gly Leu Glu Ala Lys
1               5

<210>  6
<211>  11
<212>  PRT
<213>  Artificial

<220>
<223>  an example of protease cleavage sequence

<400>  6
Arg Pro Lys Pro Val Glu Trp Arg Glu Ala Lys
1               5                   10

<210>  7
<211>  7
<212>  PRT
<213>  Artificial

<220>
<223>  PLALWAR

<400>  7
Pro Leu Ala Leu Trp Ala Arg
1               5

<210> 8
<211> 6
<212> PRT
<213> Artificial

<220>
<223> an example of protease cleavage sequence

<400> 8
Pro Leu Gly Met Trp Ser
1               5

<210> 9
<211> 5
<212> PRT
<213> Artificial

<220>
<223> an example of protease cleavage sequence

<400> 9
Pro Leu Gly Leu Gly
1               5

<210> 10
<211> 6
<212> PRT
<213> Artificial

<220>
<223> an example of protease cleavage sequence

<400> 10

Val Phe Ser Ile Pro Leu
1               5

<210>  11
<211>  5
<212>  PRT
<213>  Artificial

<220>
<223>  an example of protease cleavage sequence

<400>  11
Ile Lys Tyr His Ser
1               5

<210>  12
<211>  8
<212>  PRT
<213>  Artificial

<220>
<223>  an example of protease cleavage sequence

<400>  12
Val Pro Met Ser Met Arg Gly Gly
1               5

<210>  13
<211>  8
<212>  PRT
<213>  Artificial

<220>

<223>  an example of protease cleavage sequence

<400>  13
Arg Pro Phe Ser Met Ile Met Gly
1               5

<210>  14
<211>  8
<212>  PRT
<213>  Artificial

<220>
<223>  an example of protease cleavage sequence

<400>  14
Val Pro Leu Ser Leu Thr Met Gly
1               5

<210>  15
<211>  8
<212>  PRT
<213>  Artificial

<220>
<223>  an example of protease cleavage sequence

<400>  15
Ile Pro Glu Ser Leu Arg Ala Gly
1               5

<210>  16
<211>  7
<212>  PRT

<213>  Artificial

<220>
<223>  an example of protease cleavage sequence

<400>  16
Pro Leu Ala Tyr Trp Ala Arg
1               5

<210>  17
<211>  367
<212>  DNA
<213>  Cytomegalovirus

<400>  17
actagttatt aatagtaatc aattacgggg tcattagttc atagcccata tatggagttc      60
cgcgttacat aacttacggt aaatggcccg cctggctgac cgcccaacga cccccgccca     120
ttgacgtcaa taatgacgta tgttcccata gtaacgccaa tagggacttt ccattgacgt     180
caatgggtgg agtatttacg gtaaactgcc cacttggcag tacatcaagt gtatcatatg     240
ccaagtacgc cccctattga cgtcaatgac ggtaaatggc ccgcctggca ttatgcccag     300
tacatgacct tatgggactt tcctacttgg cagtacatct acgtattagt catcgctatt     360
accatgg                                                               367

<210>  18
<211>  1248
<212>  DNA
<213>  Gallus gallus

<400>  18
tcgaggtgag ccccacgttc tgcttcactc tccccatctc ccccccctcc ccacccccaa      60
ttttgtattt atttattttt taattatttt gtgcagcgat gggggcgggg gggggggggg     120
ggcgcgcgcc aggcggggcg gggcggggcg aggggcgggg cggggcgagg cggagaggtg     180
cggcggcagc caatcagagc ggcgcgctcc gaaagtttcc ttttatggcg aggcggcggc     240
ggcggcggcc ctataaaaag cgaagcgcgc ggcgggcggg gagtcgctgc gacgctgcct     300

tcgccccgtg ccccgctccg ccgccgcctc gcgccgcccg ccccggctct gactgaccgc 360
gttactccca caggtgagcg ggcgggacgg cccttctcct ccgggctgta attagcgctt 420
ggtttaatga cggcttgttt cttttctgtg gctgcgtgaa agccttgagg ggctccggga 480
gggccctttg tgcgggggga gcggctcggg gggtgcgtgc gtgtgtgtgt gcgtggggag 540
cgccgcgtgc ggctccgcgc tgcccggcgg ctgtgagcgc tgcgggcgcg gcgcggggct 600
ttgtgcgctc cgcagtgtgc gcgaggggag cgcggccggg ggcggtgccc cgcggtgcgg 660
ggggggctgc gaggggaaca aaggctgcgt gcggggtgtg tgcgtggggg ggtgagcagg 720
gggtgtgggc gcgtcggtcg ggctgcaacc ccccctgcac ccccctcccc gagttgctga 780
gcacggcccg gcttcgggtg cggggctccg tacggggcgt ggcgcggggc tcgccgtgcc 840
gggcgggggg tggcggcagg tgggggtgcc gggcggggcg gggccgcctc gggccgggga 900
gggctcgggg gaggggcgcg gcggcccccg gagcgccggc ggctgtcgag gcgcggcgag 960
ccgcagccat tgccttttat ggtaatcgtg cgagagggcg cagggacttc ctttgtccca 1020
aatctgtgcg gagccgaaat ctgggaggcg ccgccgcacc ccctctagcg ggcgcggggc 1080
gaagcggtgc ggcgccggca ggaaggaaat gggcggggag ggccttcgtg cgtcgccgcg 1140
ccgccgtccc cttctccctc tccagcctcg gggctgtccg cggggggacg gctgccttcg 1200
ggggggacgg ggcagggcgg ggttcggctt ctggcgtgtg accggcgg 1248

<210> 19
<211> 95
<212> DNA
<213> Oryctolagus cuniculus

<400> 19
cctctgctaa ccatgttcat gccttcttct ttttcctaca gctcctgggc aacgtgctgg 60
ttattgtgct gtctcatcat tttggcaaag aattc 95

<210> 20
<211> 1744
<212> DNA
<213> Artificial

<220>
<223> an example of CA promoter

<400> 20
actagttatt aatagtaatc aattacgggg tcattagttc atagcccata tatggagttc 60
cgcgttacat aacttacggt aaatggcccg cctggctgac cgcccaacga cccccgccca 120
ttgacgtcaa taatgacgta tgttcccata gtaacgccaa tagggacttt ccattgacgt 180
caatgggtgg agtatttacg gtaaactgcc cacttggcag tacatcaagt gtatcatatg 240
ccaagtacgc cccctattga cgtcaatgac ggtaaatggc ccgcctggca ttatgcccag 300
tacatgacct tatgggactt tcctacttgg cagtacatct acgtattagt catcgctatt 360
accatggtcg aggtgagccc cacgttctgc ttcactctcc ccatctcccc cccctcccca 420
cccccaattt tgtatttatt tattttttaa ttattttgtg cagcgatggg ggcggggggg 480
gggggggggc gcgcgccagg cggggcgggg cggggcgagg ggcggggcgg ggcgaggcgg 540
agaggtgcgg cggcagccaa tcagagcggc gcgctccgaa agtttccttt tatggcgagg 600
cggcggcggc ggcggcccta taaaaagcga agcgcgcggc gggcggggag tcgctgcgac 660
gctgccttcg ccccgtgccc cgctccgccg ccgcctcgcg ccgcccgccc cggctctgac 720
tgaccgcgtt actcccacag gtgagcgggc gggacggccc ttctcctccg ggctgtaatt 780
agcgcttggt ttaatgacgg cttgtttctt ttctgtggct gcgtgaaagc cttgaggggc 840
tccgggaggg ccctttgtgc ggggggagcg gctcgggggg tgcgtgcgtg tgtgtgtgcg 900
tggggagcgc cgcgtgcggc tccgcgctgc ccggcggctg tgagcgctgc gggcgcggcg 960
cggggctttg tgcgctccgc agtgtgcgcg aggggagcgc ggccgggggc ggtgccccgc 1020
ggtgcggggg gggctgcgag gggaacaaag gctgcgtgcg gggtgtgtgc gtgggggggt 1080
gagcaggggg tgtgggcgcg tcggtcgggc tgcaaccccc cctgcacccc cctccccgag 1140
ttgctgagca cggcccggct tcgggtgcgg ggctccgtac ggggcgtggc gcggggctcg 1200
ccgtgccggg cggggggtgg cggcaggtgg gggtgccggg cggggcgggg ccgcctcggg 1260
ccggggaggg ctcgggggag gggcgcggcg gcccccggag cgccggcggc tgtcgaggcg 1320
cggcgagccg cagccattgc cttttatggt aatcgtgcga gagggcgcag ggacttcctt 1380
tgtcccaaat ctgtgcggag ccgaaatctg ggaggcgccg ccgcaccccc tctagcgggc 1440
gcggggcgaa gcggtgcggc gccggcagga aggaaatggg cggggagggc cttcgtgcgt 1500
cgccgcgccg ccgtcccctt ctccctctcc agcctcgggg ctgtccgcgg ggggacggct 1560
gccttcgggg gggacggggc agggcggggt tcggcttctg gcgtgtgacc ggcggctcta 1620
gagcctctgc taaccatgtt catgccttct tctttttcct acagctcctg ggcaacgtgc 1680
tggttattgt gctgtctcat cattttggca aagaattcgg cttgatcgaa gcttgcccac 1740
catg 1744

<210> 21
<211> 34
<212> DNA

<213>  Bacteriophage P1

<400>  21
ataacttcgt ataatgtatg ctatacgaag ttat                                34

<210>  22
<211>  34
<212>  DNA
<213>  Saccharomyces cerevisiae

<400>  22
gaagttccta ttctctagaa agtataggaa cttc                                34

<210>  23
<211>  10
<212>  RNA
<213>  Artificial

<220>
<223>  an example of Sendai virus S sequence (w= a or c; v= a or c or g)

<400>  23
ucccwvuuwc                                                           10

<210>  24
<211>  10
<212>  RNA
<213>  Artificial

<220>
<223>  an example of Sendai virus S sequence

<400>  24

ucccaguuuc                                                                10

<210>  25
<211>  10
<212>  RNA
<213>  Artificial

<220>
<223>  an example of Sendai virus S sequence

<400>  25
ucccacuuac                                                                10

<210>  26
<211>  10
<212>  RNA
<213>  Artificial

<220>
<223>  an example of Sendai virus S sequence

<400>  26
ucccacuuuc                                                                10

<210>  27
<211>  10
<212>  DNA
<213>  Artificial

<220>
<223>  an example of Sendai virus S sequence

<400>  27

agggtcaaag                                                            10

<210>  28
<211>  10
<212>  DNA
<213>  Artificial

<220>
<223>  an example of Sendai virus S sequence

<400>  28
agggtgaatg                                                            10

<210>  29
<211>  10
<212>  DNA
<213>  Artificial

<220>
<223>  an example of Sendai virus S sequence

<400>  29
agggtgaaag                                                            10

<210>  30
<211>  9
<212>  RNA
<213>  Artificial

<220>
<223>  an example of Sendai virus E sequence

<400>  30

auucuuuuu 9

<210> 31
<211> 9
<212> DNA
<213> Artificial

<220>
<223> an example of Sendai virus E sequence

<400> 31
taagaaaaa 9

<210> 32
<211> 29
<212> DNA
<213> Artificial

<220>
<223> an artificially synthesized sequence

<400> 32
cattttggca aagaattgat taattcgag 29

<210> 33
<211> 47
<212> DNA
<213> Artificial

<220>
<223> an artificially synthesized sequence

<400> 33

tcacagcacc caagaatctc ttctggcgag caccggcatt ttgtgtc 47

<210> 34
<211> 47
<212> DNA
<213> Artificial

<220>
<223> an artificially synthesized sequence

<400> 34
gacacaaaat gccggtgctc gccagaagag attcttgggt gctgtga 47

<210> 35
<211> 42
<212> DNA
<213> Artificial

<220>
<223> an artificially synthesized sequence

<400> 35
gatcgtaatc acagtctctc gagagttgta ccatctacct ac 42

<210> 36
<211> 52
<212> DNA
<213> Artificial

<220>
<223> an artificially synthesized sequence

<400> 36

tcacagcacc gaagaatctc ctccggcgac gaccggcatt ttgtgtcgta tc 52

<210> 37
<211> 52
<212> DNA
<213> Artificial

<220>
<223> an artificially synthesized sequence

<400> 37
gatacgacac aaaatgccgg tcgtcgccgg aggagattct tcggtgctgt ga 52

<210> 38
<211> 23
<212> DNA
<213> Artificial

<220>
<223> an artificially synthesized sequence

<400> 38
aaatcctgga gtgtctttag agc 23

<210> 39
<211> 54
<212> DNA
<213> Artificial

<220>
<223> an artificially synthesized sequence

<400> 39

tctcgagtcg ctcggtacga tggccaagtt gaccagtgcc gttccggtgc tcac 54

<210> 40
<211> 85
<212> DNA
<213> Artificial

<220>
<223> an artificially synthesized sequence

<400> 40
aatgcatgat cagtaaatta caatgaacat cgaaccccag agtcccgctc agtcctgctc 60
ctcggccacg aagtgcacgc agttg 85

<210> 41
<211> 40
<212> DNA
<213> Artificial

<220>
<223> an artificially synthesized sequence

<400> 41
ccggaattca acaaatggcc gggttgttga gcaccttcga 40

<210> 42
<211> 42
<212> DNA
<213> Artificial

<220>
<223> an artificially synthesized sequence

<400> 42
ccggaattcc tagattcctc ctatcccagc tactgctgct cg 42

<210> 43
<211> 50
<212> DNA
<213> Artificial

<220>
<223> an artificially synthesized sequence

<400> 43
ctagctagcc caccatggat caagatgcct tcattctaaa agaagattct 50

<210> 44
<211> 50
<212> DNA
<213> Artificial

<220>
<223> an artificially synthesized sequence

<400> 44
ctagctagcc tagttggtca gtgactctat gtcctcttct acgagttcca 50

<210> 45
<211> 39
<212> DNA
<213> Artificial

<220>
<223> an artificially synthesized sequence

<400> 45
ggccgcgtcg acatcgatgc tagcctcgag ccgcggtac 39

<210> 46
<211> 31
<212> DNA
<213> Artificial

<220>
<223> an artificially synthesized sequence

<400> 46
cgcggctcga ggctagcatc gatgtcgacg c 31

<210> 47
<211> 22
<212> DNA
<213> Artificial

<220>
<223> an artificially synthesized sequence

<400> 47
cttaactatg cggcatcaga gc 22

<210> 48
<211> 22
<212> DNA
<213> Artificial

<220>
<223> an artificially synthesized sequence

<400> 48
gccgattcat taatgcagct gg 22

<210> 49
<211> 37
<212> DNA
<213> Artificial

<220>
<223> an artificially synthesized sequence

<400> 49
ctataggaaa ggaattccta tagtcaccaa acaagag 37

<210> 50
<211> 38
<212> DNA
<213> Artificial

<220>
<223> an artificially synthesized sequence

<400> 50
gatgagtccg tgaggacgaa actataggaa aggaattc 38

<210> 51
<211> 40
<212> DNA
<213> Artificial

<220>
<223> an artificially synthesized sequence

<400> 51
gcgggccctc tcttgtttgg tctgatgagt ccgtgaggac 40

<210> 52
<211> 9
<212> PRT
<213> Artificial

<220>
<223> an artificially synthesized sequence

<400> 52
Phe Phe Gly Ala Val Ile Gly Thr Cys
1               5

<210> 53
<211> 13
<212> PRT
<213> Artificial

<220>
<223> an artificially synthesized sequence

<400> 53
Glu Ala Arg Glu Ala Lys Arg Asp Ile Ala Leu Ile Lys
1               5                   10

<210> 54
<211> 13
<212> PRT
<213> Artificial

<220>

<223>  an artificially synthesized sequence

<400>  54
Cys Gly Thr Gly Arg Arg Pro Ile Ser Gln Asp Arg Ser
1               5                   10

## Description

### Technical Field

The present invention relates to improved methods for producing viral vectors that propagate protease-dependently.

### Background Art

Structural proteins of many viruses exhibit their activities only when processed by a protease. When such viruses are produced as recombinant viral vectors, it is necessary to propagate them in the presence of the protease. Such structural proteins include, for example, the F protein contained in the envelope of minus-strand RNA viruses. These viruses become infectious only when the F protein (F0) is cleaved to F1 and F2 by a protease derived from the host.

The Sendai virus (SeV), a minus-strand RNA virus, is expected to be an effective gene transfer vector since it has the characteristics of possessing a high gene transfer efficiency (infectivity) and ability to ensure a high expression of genes carried by the virus. In addition, the virus is thought to be highly safe as it is not pathogenic in humans and has no genetic toxicity, such as genomic integration of genes (Lamb, R.A. & Kolakofsky, D. Paramyxoviridae: the viruses and their replication. p. 1177-1204. In B. N. Fields, D. M. Knipe, and P. M. Howley (ed.), Fields virology. Lippincott-Raven, Philadelphia, Pa (1996)). Due to these reasons, the Sendai virus is being studied and developed as a "cytoplasmic gene transfer vector", which is a new type of vector different from conventional vectors. In particular, the virus is being improved into a highly safe viral vector for human gene therapy by deleting certain genes from the viral genome.

For example, in the case of gene-deficient Sendai viruses such as F gene-deficient SeV, packaging cells that supply such proteins in *trans* are required in order to harvest a high virus titer. Thus, creating the packaging cell is the most important factor. Currently, some production systems for F gene-lacking SeV vectors (SeV/ΔF: Li, H.-O. et al., J. Virol. 74, 6564-6569 (2000)), M gene-lacking SeV vectors (SeV/ΔM; Inoue, M. et al., J. Virol. 77, 6419-6429 (2003)) and such have been developed, and packaging cells capable of supplying F or M protein have been created. When the F gene which is essential for infection and fusion is deleted from the genome, the virus is modified into a nontransmissible vector because no infectious particles are released from the infected cells. When the M gene which is essential for particle formation is deleted from the genome, particle release from the infected cells is suppressed to a level lower than the detection limit. In particular, SeV/ΔF systems have become a breakthrough technology because the SeV/ΔF genome can replicate autonomously in cells introduced with SeV/ΔF, and the vector is nontransmissible while retaining the ability to express the gene within the vector at a high level.

Although SeV uses sialic acid as a receptor that exists ubiquitously on cells, its tissue tropism is very narrow in host animal bodies (rodents and such). For example, SeV efficiently propagates only in the mouse respiratory tract or chorioallantoic fluid of chicken fertile eggs. This tropism restriction results from the localization of a specific protease required for the activation of the F protein (Nagai, Y. Trends Microbiol 1, 81-87 (1993)). The blood clotting factor Xa functions as the protease in the chorioallantoic membrane of fertile eggs (Gotoh, B. et al. EMBO J 9, 4189-4195 (1990)), while tryptase Clara functions in the epithelial cells of the mouse respiratory tract (Kido, H. et al. J Biol Chem 267, 13573-13579 (1992)). The motif of the F protein (F0 protein) cleaved by these proteases is Q-S-R. Such F protein-activating proteases (cleaving F0 protein at the Q-S-R motif) are not expressed in culture systems of most cell types. Thus, a lower concentration of trypsin (7.5 µg/ml) should be added in place of natural proteases to expand SeV *in vitro* (Homma, M. & Ouchi, M. J Virol 12, 1457-1465 (1973)).

Likewise, even when preparing F gene-deficient SeV vectors(SeV/ΔF), activation of the F protein is required. Thus, it is necessary to add trypsin at the time of vector production and to conduct the culture under serum-free conditions so that trypsin can function. Such a serum-free, trypsin-present culture is very severe for cells. Most cells can be maintained for only a very short period under such conditions, and cannot serve as packaging cells. In fact, cells that can be used as packaging cells are limited to a few cell types, such as LLC-MK2, a simian kidney cell line, and BHK-21, a hamster kidney cell line. Thus, the number of cell types available for packaging cell preparations is severally limited due to such serum-free, trypsin-present culture conditions.
Non-patent Document 1: Lamb, R.A. & Kolakofsky, D. Paramyxoviridae: the viruses and their replication. p. 1177-1204. In B. N. Fields, D. M. Knipe, and P. M. Howley (ed.), Fields virology. Lippincott-Raven, Philadelphia, Pa (1996)
Non-patent Document 2: Li, H.-O. et al., J. Virol. 74, 6564-6569 (2000)
Non-patent Document 3: Inoue, M. et al., J.Virol. 77, 6419-6429 (2003)
Non-patent Document 4: Nagai, Y. Trends Microbiol 1, 81-87 (1993)
Non-patent Document 5: Gotoh, B. et al. EMBO J 9, 4189-4195 (1990)
Non-patent Document 6: Kido, H. et al. J Biol Chem 267, 13573-13579 (1992)
Non-patent Document 7: Homma, M. & Ouchi, M. J Virol 12, 1457-1465 (1973)

### Disclosure of the Invention

### Problems to Be Solved by the Invention

The present invention provides methods for producing viruses that propagate protease-dependently, using an alternative protease and independent of the original protease. The methods of the present invention enable efficient production of viral vectors.

### Means to Solve the Problems

As described above, when a protease is required to produce viruses, there were problems of reduced cell viability because of the protease added, and severe restriction of the number of cell types available for virus production. In the production of viral vectors, infectious daughter particles are generated *via* budding or release from virus-producing cells (packaging cells). In this case, the daughter particles are predicted to be contaminated with packaging cell-derived cytoplasmic components such as actin (and cell membrane components). When human gene therapy is intended, for example, when it is necessary to introduce a viral vector at a high dose, it is preferred that cytoplasmic components (and cell membrane components) contaminating the particles are derived from neither simian nor hamster cells but human cells. However, when the Sendai virus or the like is used, for example, it is necessary to use cells that can survive "under serum-free, trypsin-present conditions". Therefore, the number of cell types available as packaging cells is extremely restricted as described above, making it difficult to apply the virus to human cells. Therefore, the present inventors attempted to develop virus-producing methods with culture conditions applicable to most cell types.

A membrane bound serine endoprotease called "furin" is localized on the cell surface, and in endosomes and trans-Golgi networks within cells. Furin is ubiquitously expressed, i. e. is present in most tissues and cells, and is involved in the processing of not only cellular proteins but also membrane proteins and secretory proteins (Seidah, N.G. & Chretien, M. Brain Res. 848, 45-62 (1999), Bergeron, F. et al., J. Mol. Endocrinol. 24, 1-22 (2000), Steiner, D.F. Curr. Opin. Chem. Biol. 2 31-39 (1998)). Some viral membrane proteins are also processed by furin and the F proteins of Paramyxoviridae viruses that exhibit tropism for all organs, or F proteins of some virulent strains, often have a furin recognition sequence (Toyoda, T. et al., Virology 157, 242-247 (1987)). For example, virulent strains ofNDV (Nagai, Y et al., Virology 72, 494-5-8 (1976)), measles virus, mumps virus, HRSV, HPIV-3 (Nagai, Y. Trends Microbiol. 1, 81-87 (1993)), and the like have a furin recognition sequence. Meanwhile, SeV and HPIV-1, which are typical viruses that infect locally, have no furin recognition sequence (see Uirusu-gaku (Virology), Ed., Masakazu Hatanaka, Tokyo, Asakura Shoten, 1997, pp. 247-248).

The present inventors conceived that if the activity of furin which is ubiquitously expressed irrespective of cell type were utilized, it would become possible to produce viruses regardless of the cell line used. Specifically, if it were possible to create packaging cells introduced with an F gene into which a furin recognition sequence has been introduced, trypsin does not need to be added to the medium to activate the F protein, and therefore, a serum-free condition is not required. This removal of restrictions on culture conditions would increase options for cells that can be used as packaging cells, and particularly enable the creation of packaging cells using human cells.

The same technique can also be used to produce viral vectors of viral families other than Paramyxoviridae. For example even in other viral families, cleavage processing of the viral precursor glycoprotein by a protease is a prerequisite for the expression of membrane fusion activity. For example, human influenza virus A has a sequence involved in local infection (has no furin recognition sequence), while many viruses including the AIDS virus have a sequence involved in tropism for all organs (has a furin recognition sequence). Thus, in most viruses, the activation of the viral glycoprotein precursor by a host protease is an important factor contributing to viral tropism, and therefore, the methods of the present invention can be used to create packaging cells for various viral vectors without being limited to paramyxovirus.

Specifically, the present invention relates to methods for producing viruses that propagate protease-dependently, in a manner independent of the original protease. More specifically, the present invention relates to each of the inventions set forth in the claims. The present invention also relates to inventions comprising a desired combination of one or more (or all) inventions set forth in the claims, in particular, to inventions comprising a desired combination of one or more (or all) inventions set forth in claims (dependent claims) citing the same independent claim(s) (claim(s) relating to inventions not encompassed by inventions recited in other claims). An invention set forth in an independent claim is also intended to include any combinations of the inventions set forth in its dependent claims. Specifically, the present invention relates to:
[1] a method for producing a virus whose propagation depends on cleavage of a viral protein by a protease, wherein the method comprises the step of producing the virus in the presence of:
   (i) a modified viral protein in which a cleavage sequence for the protease is changed to a cleavage sequence for an alternative protease, and (ii) the alternative protease, and wherein the produced virus comprises the modified viral protein that is cleaved but does not comprise a gene encoding the modified viral protein;
[2] the method of [1], wherein the produced virus carries a gene encoding the viral protein comprising a wild type cleavage sequence;
[3] the method of [1], wherein the produced virus is a nontransmissible virus that lacks a gene encoding the viral protein;
[4] the method of any one of [1] to [3], wherein the alternative protease is endogenously expressed in a cell producing the virus;
[5] the method of any one of [1] to [4], wherein the alternative protease is furin;
[6] the method of any one of [1] to [5], wherein the cleavage sequence for the alternative protease comprises Arg-Xaa-Lys/Arg-Arg;
[7] the method of any one of [1] to [5], wherein the cleavage sequence for the alternative protease comprises Arg-Arg-Arg-Arg;
[8] the method of any one of [1] to [7], wherein the virus is a minus-strand RNA virus;
[9] the method of [8], wherein the minus-strand RNA virus is a Paramyxoviridae virus;
[10] the method of [8], wherein the minus-strand RNA virus is Sendai virus;
[11] a vector which encodes a modified viral protein in which a cleavage sequence for a protease of a viral protein in a virus whose propagation depends on cleavage of the viral protein by the protease is changed to a cleavage sequence for an alternative protease, wherein the vector is a viral or non-viral vector that cannot propagate in a cell producing the virus;
[12] the vector of [11], which is a plasmid;
[13] the vector of [11] or [12], wherein the expression of the modified viral protein can be induced by a recombinase;
[14] the vector of [13], wherein the recombinase is Cre or Flp;
[15] the vector of any one of [11] to [14], wherein the alternative protease is expressed endogenously in the cell producing the virus;
[16] the vector of any one of [11] to [15], wherein the alternative protease is furin;
[17] the vector of any one of [11] to [16], wherein the cleavage sequence for the alternative protease comprises Arg-Xaa-Lys/Arg-Arg;
[18] the vector of any one of [11] to [16], wherein the cleavage sequence for the alternative protease comprises Arg-Arg-Arg-Arg;
[19] the vector of any one of [11] to [18], wherein the viral protein is F protein of a minus-strand RNA virus;
[20] the vector of [19], wherein the minus-strand RNA virus is a Paramyxoviridae virus;
[21] the vector of [19], wherein the minus-strand RNA virus is Sendai virus;
[22] a mammalian cell containing the vector of any one of [11] to [21];
[23] the cell of [22], which is a cell for producing a virus whose propagation depends on cleavage of a viral protein by a protease;
[24] the cell of [22] or [23], wherein a gene encoding the modified viral protein is integrated into a chromosome of the cell;
[25] the cell of any one of [22] to [24], which is a human cell;
[26] a modified virus of a virus whose propagation depends on cleavage of a viral protein by a protease, wherein the modified virus comprises a modified viral protein in which a cleavage sequence of the viral protein for the protease is changed to a cleavage sequence for an alternative protease, and wherein the modified virus does not comprise a gene encoding the modified viral protein;
[27] the modified virus of [26], wherein a produced virus carries a gene encoding the viral protein comprising a wild type cleavage sequence;
[28] the modified virus of [26], which is a nontransmissible virus lacking a gene encoding the viral protein;
[29] the modified virus of any one of [26] to [28], wherein the alternative protease is expressed endogenously in a cell producing the virus;
[30] the modified virus of any one of [26] to [29], wherein the alternative protease is furin;
[31] the modified virus of any one of [26] to [30], wherein the cleavage sequence for the alternative protease comprises Arg-Xaa-Lys/Arg-Arg;
[32] the modified virus of any one of [26] to [30], wherein the cleavage sequence for the alternative protease comprises Arg-Arg-Arg-Arg;
[33] the modified virus of any one of [26] to [32], wherein the virus is a minus-strand RNA virus and the viral protein is F protein;
[34] the modified virus of [33], wherein the minus-strand RNA virus is a Paramyxoviridae virus; and
[35] the modified virus of [33], wherein the minus-strand RNA virus is Sendai virus.

Herein, a viral vector refers to a viral particle having infectivity, which is also a carrier for introducing a gene into a cell. "Infectivity" refers to the ability to introduce a gene carried by a viral vector into a cell when the vector is contacted with the cell. Herein, the "viral vector" includes vectors carrying, or not carrying, a foreign gene(s). The methods of the present invention for producing viruses can be used to produce both transmissible viral vectors and nontransmissible deficient-type vectors. The term "transmissible" means that when a viral vector infects a host cell, the virus is replicated in the cells to produce infectious viral particles.

"Recombinant virus" refers to a virus produced through a recombinant polynucleotide, or an amplification product thereof. "Recombinant polynucleotide" refers to a polynucleotide in which nucleotides are not linked at one end or both ends as in the natural state. Specifically, a recombinant polynucleotide is a polynucleotide in which the linkage of the polynucleotide chain has been artificially modified (cleaved and/or linked). Recombinant polynucleotides can be produced by using gene recombination methods known in the art in combination with polynucleotide synthesis, nuclease treatment, ligase treatment, etc. A recombinant virus can be produced by expressing a polynucleotide encoding a viral genome constructed through gene manipulation and virus reconstruction. For example, methods for reconstructing a virus from cDNA that encodes the viral genome are known (Y. Nagai, A. Kato, Microbiol. Immunol., 43, 613-624 (1999)).

In the present invention, "gene" refers to a genetic substance, a nucleic acid encoding a transcriptional unit. Genes may be RNAs or DNAs. In this invention, a nucleic acid encoding a protein is referred to as a gene of that protein. Further, a gene may generally not encode a protein. For example, a gene may encode a functional RNA, such as a ribozyme or antisense RNA. Generally, a gene may be a naturally-occurring or artificially-designed sequence. Furthermore, in the present invention, "DNA" includes both single-stranded and double-stranded DNAs. Moreover, "encoding a protein" means that a polynucleotide includes an ORF that encodes an amino acid sequence of the protein in a sense or antisense direction (in minus strand RNA viruses, for example), so that the protein can be expressed under appropriate conditions.

### Brief Description of the Drawings

Fig. 1 is a schematic diagram showing the sequences of the activation site in Sendai virus F protein.
Fig. 2 shows a scheme for constructing an F gene-expressing plasmid, which has a furin recognition sequence (F(furin): R-Q-K-R) at the F gene activation site.
Fig. 3 shows a scheme for constructing an F gene-expressing plasmid, which has a furin recognition sequence (F(5R): (R)-R-R-R-R) at the F gene activation site.
Fig. 4 shows the procedure for constructing pCAGGS (B type) and pCAGGS(BSX).
Fig. 5 shows the procedure for constructing pCALNdLWE-zeo-NP(Z).
Fig. 6 shows the procedure for constructing pCAGGS-P4C(-).
Fig. 7 shows the procedure for constructing pCAGGS-L(TDK).
Fig. 8 shows the procedure for constructing pCAGGS-F.
Fig. 9 shows the procedure for constructing pCAGGS-T7.
Fig. 10 shows the procedure for constructing pCAGGS-SeV and pCAGGS-SeV/ΔF-GFP.
Fig. 11 shows the procedure for constructing pCAGGS-SeV and pCAGGS-SeV/ΔF-GFP (continued from Fig. 10).
Fig. 12 shows the procedure for constructing pCAGGS-SeV (continued from Fig. 11).
Fig. 13 shows the result of examining the recovery efficiency of SeV/ΔF-GFP by a CIU assay, using varied amounts of genome DNA in the HamRbz method. The efficiency remained almost unchanged when 2 µg or more of the genome DNA was used.
Fig. 14 shows the result of examining the recovery efficiency of SeV/ΔF-GFP when the recovery was carried out by the HamRbz method using pCAGGS-F and pCAGGS-F5R. The recovery efficiency was considerably improved by using pCAGGS-F5R.
Fig. 15 is a set of photographs showing the result of an HA assay for transmissible SeV (SeV(TDK)18+GFP) recovered by the pCAGGS-T7 method. HA activity was detected only when undiluted BHK-21, BHK/T7, and 293T were inoculated into hen eggs.
Fig. 16 shows the result of examining the recovery efficiency of SeV/ΔF-GFP by a CIU assay when the amounts of genome DNA in the pCAGGS-T7 method were varied. The recovery efficiency remained almost unchanged when 0.5 to 5.0 µg of genome DNA was used, although it was the highest when 5 µg of the genome DNA was used.
Fig. 17 shows the result of examining the recovery efficiency of SeV18+GFP/ΔF by a CIU assay, when the transfer reagent used in the pCAGGS-T7 method was changed. The recovery efficiency obtained using calcium phosphate was found to be equal to or higher than that obtained using TransIT-LT-1.
Fig. 18 shows the result of examining the recovery efficiency of SeV/ΔF-GFP by CIU assay when the cell type used in the pCAGGS-T7 method was changed. Viruses were recovered from all the cell types tested. The recovery efficiency was in the order of: BHK/T7 > BHK-21 > 293T > LLC-MK2. (Note that pCAGGS-T7 was not added when BHK/T7 was used.)
Fig. 19 shows a result of Western blotting using an anti-F1 antibody to detect the F protein in viral particles of an F gene-deficient SeV vector produced from packaging cells of clone F5R2.
Fig. 20 shows the time course of the amount of infectious particles of F gene-deficient SeV vectors (SeV/ΔF-GFP) produced from packaging cells of clone F5R2 and clone F5R2-F22.
Fig. 21 shows a result of Western blotting using an anti-F1 antibody to detect the F protein in viral particles of F gene-deficient SeV vectors produced from packaging cells of clone F5R2 and clone F5R2-F22.

### Best Mode for Carrying Out the Invention

The present invention provides methods for producing viruses that propagate depending on the cleavage of a viral protein by a protease, in a manner independent of the original protease. The methods of the present invention for producing viruses comprise the step of producing viruses in the presence of an alternative protease using cells that supply a modified viral protein in *trans (i.e.,* expressed from a nucleic acid other than the produced viral genome), in which the cleavage sequence for the original protease is changed to a cleavage sequence for the alternative protease. For example, to propagate viruses whose propagation depends on a host-derived protease, the protease must normally be supplied to the viruses. However, a feature of the present invention is that a cleavage sequence in a viral protein, which serves as a substrate for the protease (tentatively referred to as "the original protease"), is changed to a cleavage sequence for an alternative protease (referred to as "the second protease"), to produce viruses using the second protease without need of the original protease.

Specifically, by producing viruses in the presence of the second protease and the modified viral protein in which the cleavage sequence for the original protease is modified into the cleavage sequence for the second protease, viruses can be propagated when the modified viral protein comprising the cleavage sequence for the second protease is cleaved and activated by the second protease. In this case, the produced viruses do not express the second protease. In other words, the produced viruses do not carry the gene encoding the second protease. Thus, a characteristic of this method is that the produced viruses cannot propagate even in the presence of the second protease unless the modified viral protein with the modified cleavage site is supplied in *trans.*

In the methods of the present invention, although it is necessary to substitute an alternative sequence for the protease cleavage sequence in the viral protein supplied in *trans* to viruses, it is not essential to modify viral genes or viral proteins of the produced viruses. Therefore, the genotypes of the produced and original viruses may be completely identical. In addition, since only the protease cleavage site needs to be different in the modified viral protein and the wild type protein, phenotypic alterations are contained to a minimum. Thus, this method is thought to have almost no influence on viral formation, propagation, gene expression, and the like. Therefore, the method of the present invention is highly versatile and may be used to produce various viruses that propagate protease-dependently.

The most preferred virus to which the methods of the present invention are applicable is a virus whose protease-dependent propagation could not be complemented by furin. Such viruses of Paramyxoviridae include, for example, Sendai virus (SeV) and human parainfluenza virus-1 (HPIV-1) which have originally no furin recognition sequences and show typical characteristics of local infection; and attenuated strains of Newcastle disease virus (NDV), HPIV-2, and such. The methods are also applicable to some virulent viral strains or viruses originally comprising a furin recognition sequence and thus exhibiting tropism for all organs. The methods of the present invention are also applicable to virulent strains of NDV and HPIV-2; and HPIV-3, mumps virus, measles virus, canine distemper virus (CDV), rinderpest virus (RPV), human respiratory syncytial virus (HRSV), and the like. Furthermore, even viral vectors other than those of Paramyxoviridae, for example, rotaviruses which belong to Reoviridae and which are thought to be activated by trypsin *via* cleavage of VP4 protein, a virion spike protein, (AriasCF, J Virol. 70(9): 5832-9 (1996), Konno T et al., Clin Infect Dis.16 Suppl 2: S92-7 (1993)), can be produced according to the present invention using a modified VP4 protein in which the trypsin cleavage site has been changed to a furin recognition sequence. In addition, the methods are also applicable to various viruses including, for example, human influenza virus A having a sequence associated with local infection (having no furin recognition sequence) and AIDS virus having a sequence involved in tropism for all organs (having a furin recognition sequence) (Nagai Y, Trends Microbiol.1, 81-87 (1993); NagaiY. Microbiol Immunol. 39, 1-9 (1995); Klenk HD, Garten W. Trends Microbiol. 2, 39-43 (1994); Lamb, R. A., and D. Kolakofsky. Paramyxoviridae: the viruses and their replication. p. 1177-1204. In B. N. Fields, D. M. Knipe, and P. M. Howley (ed.), Fields virology. Lippincott-Raven, Philadelphia, Pa. (1996); Uirusu-gaku (Virology), ed. Masakazu Hatanaka, Tokyo, Asakura Shoten, 247-248 (1997)). For example, a furin recognition sequence may be replaced with a more efficient furin recognition sequence or a recognition sequence for a protease which is expressed in virus-producing cells, other than furin.

The methods of the present invention are particularly useful when it is difficult to sufficiently supply a protease required for viral propagation to virus-producing cells. Such cases include, for example, when the protease is not expressed endogenously in virus-producing cells (or expressed at a very low level) and when the protease exerts an adverse effect on virus-producing cells. According to the methods of the present invention, viruses that originally require these proteases can be produced by using a protease expressed endogenously in the cells or a protease with low cytotoxicity. Specifically, a modified viral protein in which the protease cleavage site has been changed to a cleavage site for a protease expressed endogenously in the cells or a protease less toxic to the cells is expressed in the cells. When viruses are produced using the cells, the modified viral protein supplied by the cells is incorporated into the generated viruses and then converted into the active form by the second protease. Thus, viruses can be produced without requiring the original protease.

For example, the methods of the present invention are preferably used to produce viruses whose propagation requires a protease that is not significantly expressed endogenously in cells such as LLC-MK2 (ATCC CCL-7), Vero (ATCC CCL-81), BHK-21 (ATCC CCL-10), HEK293 ATCC CRL-1573), HT1080 (ATCC CCL-121), HeLa (ATCC CCL-2), NIH3T3 (ATCC CRL-1658), 3Y1 (JCRB 0734), COS-1 (ATCC CRL 1650), COS-7 (ATCC CRL-1651), CHO (ATCC CRL-9606), CHO-K1 (ATCC CCL 61), and derivative strains thereof, which are frequently used for producing viruses. In particular, the methods are highly useful when the protease is toxic to mammalian cells used to produce viruses. Such proteases include, for example, trypsin. Trypsin inhibits cell adhesion, and is inactivated by serum. Therefore, a serum-free culture medium must be used to allow trypsin to act on cells. Thus, the cell viability (such as survival and/or growth) is reduced under culture conditions using trypsin. Using a protease other than trypsin as the second protease can avoid cell damages caused by trypsin. Cytotoxicity of a protease can be assessed by testing whether the protease reduces cell viability as compared to when the protease is absent, under conditions that ensure protease activity required for virus production. The cell viability can be determined, for example, by an assay method based on the conversion of resazurin, a redox dye, into resorufin, a fluorescent product, by viable cells (CellTiter-Blue™ Cell Viability Assay, Promega). Alternatively, the cell viability can also be determined by LDH (lactate dehydrogenase) activity measurement, Alamar Blue fluorescence method using the redox dye Alamar Blue, the MTT or MTS method based on the conversion of a tetrazolium compound (MTT [3-(4,5-dimethylthiazol-2-yl)-2,5 tetrazolium bromide] or MTS [3-(4,5-dimethylthiazol-2-yl)-5-(3-carboxymethoxyphenyl)-2-(4-sulfophenyl)-2H-tetrazolium, inner salt]) into a formazan product by viable cells, or such.

When the cleavage site for the protease (*i.e.* the original protease) in a viral protein of these viruses is replaced with a cleavage sequence for a protease expressed endogenously in virus-producing cells or a less cytotoxic protease (*i.e.* the second protease), viruses can be produced in the absence of the originally required protease. The cleavage sequence for the second protease is not particularly limited, but is preferably a cleavage sequence for a protease expressed endogenously in, for example, the above-described mammalian cells (LLC-MK2, Vero, BHK-21, HEK293, HT1080, and derivative strains thereof), or such. A sequence that is cleaved by furin is preferably used. Since furin is ubiquitously expressed in most tissues and cells, viruses can be efficiently produced by the conversion into a furin cleavage sequence. Such furin cleavage sequences include, for example, Arg-Xaa-Lys/Arg-Arg (Xaa represents an arbitrary amino acid)). Specifically, examples of such a sequence are: RTKR, RRRR, RHKR, RQR/KR, RHKR, and RKRR. For example, when a cleavage sequence of a viral protein that originally has a sequence cleaved by trypsin or such (Xaa-Gln-Ser-Arg or Xaa-Gln-Gly-Arg) is converted into a furin cleavage sequence (Arg-Xaa-Lys/Arg-Arg), the protein can be cleaved by furin and converted into the active form.

The cleavage sequence may be converted into a cleavage sequence for a desired protease other than furin (WO 01/20989). In particular, it is preferable to convert the cleavage sequence into a cleavage sequence for a protease expressed endogenously in cells or a protease with lower cytotoxicity. Viruses can be produced more efficiently while maintaining cell viability by using genes for viral proteins having a sequence that is cleaved by such a protease. A protease that is secreted outside cells or a membrane protease which is expressed on membrane surface is preferably used to cleave envelope proteins. The protease may exist in the pathway of protein transport from translation in cells to secretion to the cell surface.

Cleavage sequences for proteases other than furin include, for example, PLGMTS (SEQ ID NO: 1) and PQGMTS (SEQ ID NO: 2) that are MMP- and collagenase-specific sequences (Japanese Patent Application No. 2002-129351). In these cases, the cleavage by MMP or collagenase occurs on the C terminal side of the third residue in the above recognition sequences. For example, it has been found that Sendai virus F protein has the F protein activity even when MTS is attached to the N-terminus of the F1 protein. In addition, it has been revealed that chymotrypsin or elastase activates a mutant SeV F protein in which R116 recognized by trypsin has been converted into I (Tashiro M et al., J Gen Virol. 73 (Pt 6): 1575-9 (1992), Itoh M, Homma M. J Gen Virol. 69 (Pt 11): 2907-11 (1988)). Hence, this sequence can be used as well. Chymotrypsin or elastase cleaves C terminal sides of hydrophobic amino acids, such as Y, F, W, L, and I. Therefore, it is expected that mutations into a sequence other than I, specifically, Y, F, W, or L, is also acceptable.

Virus-producing cells preferably express the second protease at a high level. For example, virus-producing cells can exogenously express the second protease that cleaves a modified viral protein. When the second protease is expressed at a high level, the cleavage of the modified viral protein can be enhanced to further improve the efficiency of virus production. To achieve this, an expression vector encoding the second protease is introduced into cells. A desired promoter for expressing genes in mammalian cells can be used to express the second protease. In addition, such an expression vector may be constructed using a recombinase target sequence or inducible promoter so that the expression of the second protease is induced depending on a stimulation (described below).

Examples of the second protease also includes calpain, ubiquitin-proteasome system, neprilysin, MMP, serine protease, and aminopeptidase. Calpain is a protease that is activated upon binding with calcium. Cleavage sites of proteins such as alpha-actinin, troponin, and connectin can be used for the cleavage sequence of calpain. Calpain cleavage sequences (Karlsson, J.O. et al. (2000) Cell Biol. Int. 24, 235-243) include, for example, Leu-Leu-Val-Tyr.

Alternatively, it is possible to use cleavage sequences for MMP that degrades extracellular matrix (ECM) or ADAM (a disintegrin and metalloproteinase) of the gene family related to MMP. The cleavage sequence in cartilage proteoglycan (aggrecan) degraded by ADAMTS (ADAM with thrombospondin motif), which is essential for the degradation of aggrecan is known (Tortorella, M.D. et al. (2000) J. Biol. Chem. 275, 18566-18573). These protease recognition sequences can be used to produce viral vectors using the proteases.

ECM-degrading serine proteases include, for example, cathepsin G, elastase, plasmin, plasminogen activator (PA), tumor trypsin, chymotrypsin-like neutral protease, and thrombin. Plasmin is generated *via* limited proteolysis of plasminogen which is present in the living body in the inactive state. PA includes tissue PA (tPA) involved in blood clotting and urokinase PA (uPA) involved in ECM degradation (Blasi, F. and Verde, P. Semin. Cancer Bio. 1:117-126, 1990). Cleavage sequences for uPA and tPA are well known (Rijken, D.C. et al. (1982) J. Biol. Chem. 257, 2920-2925; Wallen, P. et al. (1982) Biochim. Biophys. Acta 719, 318-328; Tate, K.M. et al. (1987) Biochemistry 26, 338-343). The substrate sequences generally used are VGR (Dooijewaard, G, and KLUFT, C. (1983) Adv. Exp. Med. Biol. 156, 115-120) and Substrate S-2288 (Ile-Pro-Arg; Matsuo, O. et al. (1983) Jpn. J. Physiol. 33, 1031-1037). Butenas provided highly specific sequences for tPA using 54 types of fluorescent substrates (Butenas, S. et al. (1997) Biochemistry 36, 2123-2131). tPA exhibited strong degradation activity to FPR and VPR. Therefore, these sequences are particularly preferably used. Plasmin degrades fibronectin, tenascin, laminin, and the like.

In addition, ECM proteases that are categorized into cysteine proteases or aspartic proteases are also known. Specifically, such proteases include, for example, cathepsin B (Sloane, B.F., Semin. Cancer Biol. 1:137-152, 1990) whose substrates include laminin, proteoglycan, fibronectin, collagen, and pro-collagenase (activated by proteolysis); cathepsin L (Kane, S.E. and Gottesman, M.M., Semin. Cancer Biol. 1:127-136, 1990) whose substrates include elastin, proteoglycan, fibronectin, laminin, and elastase (activation); and cathepsin D (Rochefort, H., Semin. Cancer Biol. 1:153-160, 1990) whose substrates include laminin, fibronectin, proteoglycan, and cathepsin B and L (activation).

Metalloproteinases are metalloenzymes containing metal atoms, such as Zn, and are reported to include, for example, caspase, aminopeptidase, angiotensin I converting enzyme, and collagenase. More than 16 types of matrix metalloproteinases (MMP) are reported as ECM-degrading metalloproteinases. Representative MMPs include, for example, collagenase-1, -2, and -3 (MMP-1, -8, and -13), gelatinase A and B (MMP-2 and -9), stromelysin 1, 2, and 3 (MMP-3, -10, and -11), matrilysin (MMP-7), and membrane metalloproteinases (MT1-MMP and MT2-MMP). Furthermore, ECM-degrading metalloproteinases also include aminopeptidase, for example, aminopeptidase N/CD13 and aminopeptidase B which degrade ECM-constituting proteins and such.

Among them, collagenases (MMP-1, -8, and -13) cleave type I, II, and III collagen molecules, which are fibrous collagens, at specific sites. There are two types of known gelatinases, namely gelatinase A (MMP-2) and gelatinase B (MMP-9). Gelatinases, which are also called "type IV collagenases", degrade type IV collagen which is a major component of basal membrane. Gelatinases also degrade type V collagen and elastin. It is also known that MMP-2 cleaves type I collagen at the same site as MMP-1. MMP-9 does not degrade laminin and fibronectin, while MMP-2 does. Stromelysins (MMP-3 and -10) have a broad substrate specificity, and degrade proteoglycan, type III, IV, and IX collagens, laminin, and fibronectin. Matrilysin (MMP-7) is a molecule having no hemopexin domain, and its substrate specificity is identical to that of MMP-3. Particularly, matrilysin exhibits strong degradation activity to proteoglycan and elastin. Membrane-type MMPs (MT-MMPs; MT1-, MT2-, MT3-, MT4-, MT5-, and MT6-MMPs) are MMPs having a transmembrane structure. MT-MMPs have an insertion sequence (about 10 amino acids) between the propeptide domain and active site. This insertion sequence comprises Arg-Xaa-Lys-Arg (Xaa represents an arbitrary amino acid), and is cleaved by intracellular processing during the process of transport to the plasma membrane, thereby activating MT-MMPs. Known MT-MMPs include MT1-MMP (MMP-14), MT2-MMP (MMP-15), MT3-MMP (MMP-16), MT4-MMP (MMP-17), MT5-MMP (MMP-23), and MT-6-MMP (MMP-25). For example, MT1-MMP degrades type I, II, and III collagens, while MT3-MMP degrades type III collagen.

Many cleavage substrates for MMP are known. Substrate sequences degraded by MMPs in general include PLGLWAR (SEQ ID NO: 3) (Bickett, D. M. et al. (1993) Anal. Biochem. 212, 58-64), GPLGMRGL (SEQ ID NO: 4) (Deng, S.J. et al. (2000) J. Biol. Chem. 275, 31422-31427), PQGLEAK (SEQ ID NO: 5) (Beekman, B. et al. (1996) FEBS Lett. 390, 221-225), RPKPVEWREAK (SEQ ID NO: 6) (Beekman, B. et al. (1997) FEBS Lett. 418, 305-309), and PLALWAR (SEQ ID NO: 7) (Jacobsen, E.J. et al. (1999) J. Med. Chem. 42, 1525-1536). Proteolytic substrates for MMP-2 and -9 include PLGMWS (SEQ ID NO: 8) (Netzel-Arnett, S. et al. (1991) Anal. Biochem. 195, 86-92) and PLGLG (SEQ ID NO: 9) (Weingarten, H. et al. (1985) Biochemistry 24, 6730-6734).

Recently, proteolytic substrate sequences for MMP9 (Kridel, S.J. et al. (2001) J. Biol. Chem. 276, 20572-20578), MMP2 (Chen, E.I. et al. (2002) J. Biol. Chem. 277, 4485-4491), and MT1-MMP (Kridel, S.J. et al. (2002) J. Biol. Chem. In JBC Papers in Press, April 16, 2002, Manuscript M111574200) have been identified by phage-displayed peptide library screening. The amino acid sequences specified in these reports have been categorized into four groups based on the proteolytic activities of the three MMPs. It has been shown that the sequences of Group IV are cleaved specifically by MT1-MMP and the sequences VFSIPL (SEQ ID NO: 10) and IKYHS (SEQ ID NO: 11) which contain no Arg are substrates that are degraded by only MT-MMP but not by MMP9 nor MMP2.

For example, MMP9 cleavage sequences include, for example, Pro-X-X-Hy (X represents an arbitrary residue, while Hy represents a hydrophobic residue). In particular, Pro-X-X-Hy-(Ser/Thr) is preferred. A more specific example is Pro-Arg-(Ser/Thr)-Hy-(Ser/Thr) (cleavage takes place between X and Hy). Hy (hydrophobic residue) includes, for example, Leu, Val, Tyr, Ile, Phe, Trp, and Met, but is not limited thereto. Alternatively, other cleavage sequences have been identified (see, for example, Group I, II, IIIA, and IIIB described in the following document: Kridel, S.J. et al. (2001) J. Biol. Chem. 276, 20572-20578), and a desired sequence of these can be used. Meanwhile, the MMP2 cleavage sequence may be Pro-X-X-Hy described above, and in addition includes (Ile/Leu)-X-X-Hy, Hy-Ser-X-Leu, and His-X-X-Hy (see, for example, Group I, II, III, and IV described Chen, E.I. et al. (2002) J. Biol. Chem. 277, 4485-4491). The cleavage sequences for the MMP family including MMP-7, MMP-1, MMP-2, MMP-9, MMP-3, and MT1-MMP (MMP-14) can be appropriately selected, for example, by referring to sequences of natural substrate proteins, by screening peptide libraries, or such (Turk, B.E. et al., Nature Biotech. 19, 661-667 (2001); Woessner, J.F. and Nagase, H. Matrix metalloproteinases and TIMPs. (Oxford University Press, Oxford, UK, 2000); Fernandez-Patron, C. et al., Circ. Res. 85: 906-911, 1999; Nakamura, H. et al., J. Biol. Chem. 275: 38885-38890,2000; McQuibban, G.A. et al., Science 289: 1202-1206, 2000; Sasaki, T. et al., J. Biol. Chem. 272: 9237-9243, 1997). For example, examples of 8 amino acids P4-P3-P2-P1-P1'-P2'-P3'-P4' (cleaved between P1 and P1') at the cleavage site include, but are not limited to: VPMS-MRGG (SEQ ID NO: 12) for MMP-1; RPFS-MIMG (SEQ ID NO: 13) for MMP-3; VPLS-LTMG (SEQ ID NO: 14) for MMP-7; and IPES-LRAG (SEQ ID NO: 15) for MT1-MMP. Such sequences for MMP-8 include, for example, PLAYWAR (SEQ ID NO: 16) (Nezel-Amett, S. et al., Anal. Biochem. 195: 86, 1991). Various synthetic substrates for MMPs are available, and their sequences may be compared with each other (see, for example, sections of "MMP Substrate" in Calbiochem® catalog, Merk).

The protease for cleaving the viral protein is changed by replacing the sequence of protease cleavage site in a viral protein with a cleavage sequence for any one of the proteases described above. Western blotting for the modified protein expressed in cells can examine whether the modified viral protein is cleaved efficiently by the second protease. Alternatively, it can also be confirmed by detecting the function of viral proteins activated by cleavage (PCT/JP03/05528). For example, when the viral protein has the activity of fusing with the cell membrane, cells are transfected with a plasmid vector expressing the modified viral protein and cultured in the presence of the second protease and syncytial formation is detected. This detection enables the confirmation of modified viral protein activation *via* cleavage by the second protease.

The protein with a modified cleavage site is not encoded by the genome of virus produced according to the present invention. Such viruses include viruses carrying the gene encoding a viral protein having the original cleavage sequence prior to cleavage sequence modification. Specifically, even though these viruses comprise the modified viral protein, the viral genomes comprise the gene encoding the viral protein having the wild type cleavage sequence. Thus, the viruses propagate depending on the protease that cleaves the wild type cleavage sequence (when the virus has viral genes required for viral propagation). To produce viruses carrying a viral gene having the wild type cleavage sequence, in addition to expressing the modified viral protein in virus-producing cells in the presence of the second protease, a protease (specifically, the original protease) that cleaves the wild type cleavage sequence may be added to or expressed in the virus-producing cells. Use of the modified viral protein is expected to increase the amount of virus production (see Example 5).

In an embodiment of the present invention, the virus to be produced is a deficient virus that lacks a viral gene having a cleavage sequence to be modified. Since the virus lacks the viral gene, the functional viral protein is not expressed in the target cells infected with the virus, and thus the virus cannot propagate (regardless of the presence of the protease). Specifically, this virus is nontransmittable. The method of the present invention particularly has the advantage of enabling efficient production of nontransmissible deficient-type vectors that are generally difficult to produce in high titers, in various cell types including human cells.

When viruses are produced according to the present invention, a modified viral protein in which the cleavage site has been converted into a cleavage sequence for a second protease is supplied in *trans* (*i.e.* from other than viral genome) as described above. For this purpose, a vector expressing the modified viral protein is separately constructed, introduced into virus-producing cells, and expressed in the cells. The expression vector for the modified viral protein may be a desired vector. When a viral vector is used, as a matter of course, a viral vector that cannot propagate in virus-producing cells must be used to avoid contamination of the produced viruses by the vector. Furthermore, a viral vector of a different species is used to avoid the incorporation of the gene for the modified protein into the produced viruses. Alternatively, when using the same viral species, the signal sequence required for the incorporation into the virus is removed. It is preferable to use, for example, a viral vector whose propagation ability has been inactivated, a nontransmittable viral vector of a species different from the virus to be produced, or a non-viral vector. Specifically, replication-deficient viral vectors derived from different species and plasmid vectors are preferred.

More preferable vectors for expressing modified viral proteins include plasmid vectors. Methods for transfecting cells with plasmids include the calcium phosphate method (Graham, F. L. and Van Der Eb, J., 1973, Virology 52: 456; Wigler, M. and Silverstein, S., 1977, Cell 11: 223), methods using various transfection reagents, electroporation, or such. The calcium phosphate method can be performed, for example, under the conditions of 2 to 4% CO₂, 20 to 30 µg/ml of DNA concentration in the precipitate mixture, and for 15 to 24 hours at 35°C, according to Chen and Okayama (Chen, C. and Okayama, H., 1987, Mol. Cell. Biol. 7: 2745). As a transfection reagent, DEAE-dextran (Sigma #D-9885 M.W. 5x 10⁵), DOTMA (Roche), Superfect (QIAGEN #301305), DOTAP, DOPE, DOSPER (Roche #1811169), TransIT-LT1 (Mirus, Product No. MIR 2300), or the like can be used. To prevent transfection reagent/DNA complexes from decomposing in endosomes, chloroquine may also be added (Calos, M. P., 1983, Proc. Natl. Acad. Sci. USA 80: 3015). Electroporation has a wide versatility because it is not cell-selective. It is applied by optimizing the duration of pulse electric current, shape of the pulse, potency of electric field (gap between electrodes, voltage), conductivity of buffer, DNA concentration, and cell density. The methods using transfection reagents are suitable for introducing DNA into cells for vector reconstitution, since they are simple to operate and facilitate examination of many samples using a large amount of cells. Preferably, the Superfect Transfection Reagent (QIAGEN, Cat No. 301305), the DOSPER Liposomal Transfection Reagent (Roche, Cat No. 1811169), TransIT-LT1 (Mirus, Product No. MIR 2300) or such is used; however, the transfection reagents are not limited to these.

A desired promoter for expression in mammalian cells may be used to express the modified protein. Such promoters include, for example, cytomegalovirus (CMV) promoter, LTR promoter of Rous sarcoma virus, thymidine kinase (TK) promoter, α- and β-actin promoters, SV40 early gene promoter, EF1α promoter, and SRα promoter, and hybrid promoters of these promoters (Kim DW et al., 1990, Use of the human elongation factor 1 alpha promoter as a versatile and efficient expression system. Gene 91(2):217-23; Chapman BS et al., 1991, Effect of intron A from human cytomegalovirus (Towne) immediate-early gene on heterologous expression in mammalian cells, Nucleic Acids Res. 14: 3979-3986; Takebe Y et al., 1988, SR alpha promoter: an efficient and versatile mammalian cDNA expression system composed of the simian virus 40 early promoter and the R-U5 segment of human T-cell leukemia virus type 1 long terminal repeat. Mol. Cell Biol. 1: 466-472).

Particularly preferable promoters that are used to express modified proteins include a promoter comprising a cytomegalovirus enhancer and a chicken β-actin promoter (referred to as CA promoter). A CA promoter refers to a hybrid promoter comprising (i) an enhancer sequence of a cytomegalovirus (CMV) immediate early (IE) gene and (ii) a promoter sequence of a chicken β-actin gene. High titer viruses can be produced by expressing modified viral proteins using a CA promoter. For the CMV IE enhancer, the enhancer of an immediately early gene from a desired CMV strain can be used, for example, DNA comprising the nucleotide sequence of SEQ ID NO: 17.

The chicken β-actin promoter includes a DNA fragment with promoter activity that comprises a transcription initiation site for the genomic DNA of the chicken β-actin gene and a TATA box (Ann. Rev. Biochem. 50, 349-383, 1981) and CCAAT box (Nucl. Acids Res. 8, 127-142, 1980). The nucleotide sequence of the chicken β-actin gene promoter has been reported by, for example, T. A. Kost et al. (Nucl. Acids Res. 11, 8287-8286, 1983). In the chicken β-actin promoter, the region from G (guanine) at position -909 to G (guanine) at position -7 upstream of the translation initiation codon (ATG) of the original β-actin structural gene is considered as an intron. Since this intron has transcription-promoting activity, it is preferable to use a genomic DNA fragment comprising at least a portion of this intron. Specifically, examples of such chicken β-actin promoters include, for example, DNA comprising the nucleotide sequence of SEQ ID NO: 18. For the intron acceptor sequence, an intron acceptor sequence from a different gene can be used. For example, a splicing acceptor sequence of rabbit β-globin may be used. More specifically, the acceptor site of the second intron, which is located immediately before the initiation codon of rabbit β-globin, can be used. Specifically, such acceptor sequences include, for example, DNA comprising the nucleotide sequence of SEQ ID NO: 19. A CA promoter of the present invention is preferably a DNA in which a chicken β-actin promoter comprising a portion of the intron is linked downstream of a CMV IE enhancer sequence and a desired intron acceptor sequence is added downstream thereof. An example is shown in SEQ ID NO: 20. To express a protein, the last ATG in this sequence is used as the start codon and the coding sequence for the modified viral protein may be linked thereto.

The CMV enhancer sequence and chicken β-actin gene promoter, which are used as the CA promoter, vary in their sequences depending on the strains or individuals from which the sequences are isolated. These sequences may be slightly modified so that restriction enzyme recognition sites can be added or deleted, or linker sequences can be inserted (Molecular cloning: a laboratory manual., 3rd ed., Joseph Sambrook, David W. Russell., Cold Spring Harbor Laboratory Press, 2001). Specifically, the sequences may not be completely identical to the exemplary sequence shown in SEQ ID NO: 20. Such sequences can be suitably used as long as they have an equivalent or higher (for example, 70% or higher, preferably 80% or higher, 90% or higher, or 100% or higher) promoter activity. For variants of the CMV enhancer sequence and chicken β-actin gene promoter sequence, for example, the sequences of GenBank accession numbers AF334827, AY237157, AJ575208, X00182, and such can be used. To identify from the above sequences those that are required for constructing a CA promoter, the sequences may be aligned with SEQ ID NOs: 17 and 18 and matched regions may be selected from the alignments. DNA excised from pCAGGS (Niwa, H. et al. (1991) Gene. 108: 193-199, Japanese Patent Application Kokai Publication No. (JP-A) H3-168087 (unexamined, published Japanese patent application) or pCALNdLw (Arai, T. et al. J. Virology 72, 1998, p1115-1121) can be used to construct a CA promoter.

Variants of the CMV IE enhancer sequence and chicken β-actin promoter as described above include sequences that have equivalent promoter activity, and which comprise a nucleotide sequence having a substitution, deletion, and/or insertion of 30% or less, preferably 20% or less, more preferably 15% or less, more preferably 10% or less, more preferably 5% or less, more preferably 3% or less of the nucleotides in the CMV IE enhancer sequence of SEQ ID NO: 17 and the chicken β-actin promoter of SEQ ID NO: 18. These sequences exhibit high homology to the nucleotide sequence of either SEQ ID NO: 17 or 18. High homology nucleotide sequences include those with an identity of, for example, 70% or higher, more preferably 75% or higher, even more preferably 80% or higher, still more preferably 85% or higher, yet more preferably 90% or higher, even still more preferably 93% or higher, yet still more preferably 95% or higher, yet still even more preferably 96% or higher. The nucleotide sequence identity can be determined, for example, using the BLAST program (Altschul, S. F. et al., 1990, J. Mol. Biol. 215: 403-410). For example, search is carried out on the BLAST web page ofNCBI (National Center for Biotechnology Information) using default parameters, with all the filters including Low Complexity turned off(Altschul, S. F. et al. (1993) Nature Genet. 3:266-272; Madden, T. L. et al. (1996) Meth. Enzymol. 266:131-141; Altschul, S. F. et al. (1997) Nucleic Acids Res. 25:3389-3402; Zhang, J. & Madden, T. L. (1997) Genome Res. 7:649-656). Sequence identity can be determined, for example, by comparing two sequences using the blast2sequences program to prepare an alignment of the two sequences (Tatiana A et al. (1999) FEMS Microbiol Lett. 174:247-250). Gaps are treated in the same way as mismatches. For example, an identity score is calculated in view of the entire nucleotide sequences of SEQ ID NOs: 17 and 18. Specifically, the ratio of the number of identical nucleotides in the alignment to the total number of nucleotides of SEQ ID NO: 17 or 18 is calculated. Gaps outside of SEQ ID NO: 1 or 2 in the alignment is excluded from the calculation.

The CMV enhancer sequence and chicken β-actin promoter sequence can also be isolated by hybridization from the nucleic acid of a CMV genome and chicken genomic DNA, respectively. The CMV enhancer and chicken β-actin promoter used in the present invention may be DNAs that have an equivalent promoter activity and hybridize under stringent conditions to the nucleotide sequences of SEQ ID NOs: 17 and 18, respectively, or to the complementary sequences thereof. When hybridization is used, such a promoter can be identified, for example, by preparing a probe either from the nucleotide sequence of SEQ ID NO: 17 or 18 or the complementary sequence thereof, or from a DNA to be hybridized, and then detecting whether the probe hybridizes to the other DNA. Stringent hybridization conditions are, for example, hybridization at 60°C, preferably at 65°C, more preferably at 68°C in a solution containing 5x SSC, 7%(W/V) SDS, 100 µg/ml denatured salmon sperm DNA, and 5x Denhardt's solution (1x Denhardt's solution contains 0.2% polyvinylpyrrolidone, 0.2% bovine serum albumin, and 0.2% Ficoll); and washing twice in 2x SSC, preferably 1x SSC, more preferably 0.5x SSC, still more preferably 0.1x SSC, at the same temperature as the hybridization temperature.

When an expression system inducible in response to a particular stimulus is used for a vector for expressing the modified viral protein, the expression of the modified viral protein can be induced specifically at the time of virus production. Since viral proteins sometimes exhibit cytotoxicity, the use of such an inducible expression system is advantageous. To achieve this purpose, it is possible to use, for example, a system using the *E. coli* tetracycline resistance operon (Gossen, M., et al. (1995) Science 268:1766-1769; Tet Expression Systems and Cell Lines (July 1996) CLONTECHniques XI(3):2-5), the ecdysone inducible expression system using ecdysone receptor, RXR, and ecdysone receptor response sequence (pVgRXR, pIND; Ecdysone-inducible Mammalian Expression Kit, Invitrogen), or a system using a sequence-specific recombinase, such as Cre/loxP or FLP/FRT. In particular, an inducible expression system based on a sequence-specific recombinase is suitable for the expression system for the modified viral protein, since this system is easy to use in combination with various promoters and allows a very strict on/off control of expression.

Cre is an approximately 38 kDa cyclization recombinase carried by bacteriophage P1 and performs site-specific DNA recombination between *loxP* sites (Sauer B, Henderson N. 1988. Site-specific DNA recombination in mammalian cells by the Cre recombinase of bacteriophage P1. Proc Natl Acad Sci USA 85:5166-70; Sternberg N, Hamilton D. 1981. Bacteriophage P1 site-specific recombination. I. Recombination between loxP sites. J Mol Biol 150:467-86; Brian Sauer, Methods of Enzymology; 1993, Vol. 225, 890-900; Nagy A. 2000. Cre recombinase: the universal reagent for genome tailoring. Genesis 26:99-109). loxP is a 13-bp asymmetric inverted repeat sequence which comprises an 8-bp spacer (ATAACTTCGTATAATGTATGCTATACGAAGTTAT; the underlines indicate the inverted repeats; SEQ ID NO: 21). Cre/loxP inducible expression plasmid pCALNdlw (Arai, T. et al., J. Virology 72, 1998, p1115-1121) can be used to construct vectors whose expression can be induced by Cre. To express Cre, for example, cells are infected with adenovirus AxCANCre for example, at an moi of 3 to 5 by the method of Saito et al. (Saito et al., Nucl. Acids Res. 23: 3816-3821 (1995); Arai, T.et al., J. Virol 72,1115-1121 (1998)).

FLP recombinase is a flippase recombinase of about 49 kDa derived from the 2 micron plasmid of yeast *Saccharomyces cerevisiae* and targets the FLP recombinase target (FRT) sequence for recombination (Utomo AR, Nikitin AY, Lee WH. 1999. Temporal, spatial, and cell type-specific control of Cre-mediated DNA recombination in transgenic mice. Nat Biotechnol 17:1091-6; Broach, J. R., Guarascio, V. R. & Jayaram, M. (1982) Cell 29, 227-34; Cox, M. M. (1983) Proc. Natl. Acad. Sci. USA 80, 4223-227; Vetter, D., Andrews, B. J., Roberts-Beatty, L. & Sadowski, P. D. (1983) Proc. Natl. Acad. Sci. USA 80, 7284-288; Abremski, K. & Hoess, R. (1984) J. Biol. Chem. 259, 1509-514; Stark, W. M., Boocock, M. R. & Sherratt, D. J. (1992) Trends Genet. 8, 432-39; Kilby, N. J., Snaith, M. R. & Murray, J. A. H. (1993) Trends Genet. 9, 413-21). Like loxP, FRT sequences also consist of a 13-bp repeat sequence comprising an 8-bp spacer (GAAGTTCCTATTCTCTAGAAAGTATAGGAACTTC; SEQ ID NO: 22) (Andrews, B. J. et al. (1985). The FLP Recombinase of the 2 Micron Circle DNA of Yeast: Interaction with its Target Sequences. Cell 40, 795-803). In addition, target-specific recombination can be achieved using mutant sequences of the loxP site and FRT site described above (Baszczynski, Christopher L. et al, US Patent Application 20040003435).

To construct a vector whose expression is induced in a recombination enzyme-dependent manner, a DNA fragment flanked by a pair of target sequences of the recombination enzyme is inserted between a promoter and the coding sequence of a modified viral protein. In this state, due to interference by the inserted DNA fragment, the modified viral protein is not expressed. However, when the recombination enzyme acts on the DNA, the target sequence-flanked DNA is excised, which enables the recombination enzyme to be expressed from the promoter. As described above, expression from the promoter can be induced by a recombination enzyme. The DNA flanked by the recombination enzyme target sequences is preferably designed to contain a transcription termination signal and/or stop codon so that the expression of the downstream gene is definitely inhibited in the absence of the recombination enzyme action. An appropriate marker gene can also be inserted into the DNA flanked by the target sequences of the recombination enzyme.

The expression vector for the modified viral protein is expressed by introducing it into mammalian cells by an appropriate method, such as transfection or infection, depending on the vector type. Various transfection reagents can be used for transfection. For example, it is preferable to use cationic lipids, such as DOTMA (Roche), Superfect (QIAGEN #301305), DOTAP, DOPE, DOSPER (Roche #1811169), and TransIT-LT1 (Mirus, Product No. MIR 2300). The expression vector for the modified viral protein may be transiently introduced into virus-producing cells at the time of virus production and episomally expressed in them or may be introduced into chromosomes of virus-producing cells to establish stable transformants. Cells stably introduced with the modified viral protein expression vector are useful as helper cells to produce viruses. Such stable transformants can be obtained by inserting a drug resistance marker gene into the vector and selecting drug resistant clones. Western blotting is preferably carried out to isolate cell clones expressing the modified viral protein at high levels. In addition, the cell clones are actually allowed to produce viral vectors to select cells capable of producing high titer viruses.

Cells to be introduced with the modified viral protein expression vector are not specifically limited as long as they are mammalian cells which allow viral propagation. For example, mouse, rat, monkey, and human derived cell lines can be used. Specifically, such cell lines include, for example, NIH3T3, 3Y1, LLC-MK2, Vero, CV-1, HeLa, HEK293, COS-1, COS-7, CHO, CHO-K1, HT1080, and derivative lines thereof. When viral vectors are produced for the purpose of introducing genes into human cells, human cells are preferably used to produce the viruses. This is because daughter viral particles released from virus-producing cells can be contaminated with cytoplasmic and plasma membrane components derived from the virus-producing cells, as described above. Therefore, when non-human cells are used to produce viruses, such components possibly produce adverse effects on humans or induce immune responses. Such problems can be overcome by producing viruses using cells from the same species as the subject to be introduced with the viral vector.

Cell lines that can express the modified viral protein expression vector are useful to produce not only viruses of the same species from which the protein is derived, but also viruses of a different species, which has been pseudotyped with the modified viral protein. For example, adenoviral vectors pseudotyped with a modified F protein of minus-strand RNA virus can also be constructed by producing adenoviruses in the presence of the modified F protein and HN protein of minus-strand RNA virus (Galanis, E. et al., Hum. Gene Ther. 12, 811-821 (2001)). Furthermore, for example, the cells can be used to pseudotype retroviruses with the modified F protein and HN protein (Spiegel, M. et al., J Virol. 72(6) 5296-5302 (1998)). It is possible to further introduce a vector expressing other viral proteins and/or viral genomic RNA into the cells, depending on the type of virus to be produced.

Viral vectors comprising a modified viral protein can be produced by using the cells described above (and a protease that cleaves the modified viral protein) in the step of producing each virus. Specifically, the genomic RNA of a viral vector is transcribed in cells expressing the modified viral protein in the presence of a protease that cleaves the modified viral protein. When a viral protein needs to be supplied in *trans* to form viral particles, the viral protein is also expressed. The transcribed viral genomic RNA forms a complex with viral proteins, thus forming viral particles into which the modified viral protein has been incorporated. Viral vectors comprising the modified viral protein can be prepared by harvesting the viral particles from the culture supernatant or cells.

The method of the present invention can be applied to protease-dependent production of a desired viral vector. As an example, a method for producing a minus-strand RNA viral vector is more specifically described below. "Minus-strand RNA virus" refers to viruses that contain a minus strand (an antisense strand complementary to a sense strand encoding viral proteins) RNA as the genome. The minus-strand RNA is also referred to as negative strand RNA. Minus-strand RNA viruses are excellent gene transfer vectors. They do not have a DNA phase and carry out transcription and replication only in the host cytoplasm, and consequently, chromosomal integration does not occur (Lamb, R.A. and Kolakofsky, D., Paramyxoviridae: The viruses and their replication. In: Fields BN, Knipe DM, Howley PM, (eds). Fields of Virology. Vol. 2. Lippincott - Raven Publishers: Philadelphia, 1996, pp. 1177-1204). Therefore, safety issues such as transformation and immortalization due to chromosomal aberration do not occur. This characteristic of minus-strand RNA viruses contributes greatly to safety when the viruses are used as vectors. For example, results on foreign gene expression show that even after multiple continuous passages of SeV, which is one of the minus-strand RNA viruses, almost no nucleotide mutation is observed. This suggests that the viral genome is highly stable and the inserted foreign genes are stably expressed over long periods of time (Yu, D. et al., Genes Cells 2, 457-466 (1997)).

Further, there are qualitative advantages associated with SeV not having a capsid structural protein, such as packaging flexibility and insert gene size, suggesting that minus-strand RNA viral vectors may become a novel class of highly efficient vectors for human gene therapy.

The minus-strand RNA virus used in the present invention particularly includes single-stranded minus-strand RNA viruses (also referred to as non-segmented minus-strand RNA viruses), which have a single-stranded negative strand [*i.e.,* a minus strand] RNA as the genome. Such viruses include viruses belonging to Paramyxoviridae (including the genera *Paramyxovirus, Morbillivirus,* and *Rubulavirus*), Rhabdoviridae (including the genera *Vesiculovirus, Lyssavirus,* and *Ephemerovirus),* Filoviridae, Orthomyxoviridae, (including Influenza viruses A, B, and C, and Thogoto-like viruses), Bunyaviridae (including the genera *Bunyavirus, Hantavirus, Nairovirus,* and *Phlebovirus*), Arenaviridae, and the like.

More specific examples of virus that may be used in the context of the present invention include those selected from the group consisting of: Sendai virus (SeV), human parainfluenza virus-1 (HPIV-1), human parainfluenza virus-3 (HPIV-3), phocine distemper virus (PDV), canine distemper virus (CDV), dolphin molbillivirus (DMV), peste-des-petits-ruminants virus (PDPR), measles virus (MV), rinderpest virus (RPV), Hendra virus (Hendra), Nipah virus (Nipah), human parainfluenza virus-2 (HPIV-2), simian parainfluenza virus 5 (SV5), human parainfluenza virus-4a (HPIV-4a), human parainfluenza virus-4b (HPIV-4b), mumps virus (Mumps), and Newcastle disease virus (NDV).

Major targets of the methods of the present invention for producing viruses are viruses whose propagation requires a protease other than furin. However, for example, even in the case of viruses originally comprising a furin-type cleaving sequence in the F protein, a furin cleavable F protein may be supplied in *trans* from virus-producing cells when an attenuated strain having a mutant F protein with a modified cleavage site is produced.

When the cleavage site of minus-strand RNA virus F protein is modified, the cleavage sequence is preferably designed so that the N-terminus of F1 fragment after cleavage is identical to the N-terminus of F1 fragment of wild type F protein. When a linker is inserted into the cleavage site to ensure an efficient cleavage reaction, the cleavage sequence is preferably designed so that minimal amino acids are attached to the N-terminus of F1 fragment after cleavage as compared to that of wild type F1. For example, the cleavage sequence is designed so that the N-terminus after cleavage is attached with 5 amino acids or less, preferably 4 amino acids or less, more preferably 3 amino acids or less (for example, 1, 2, or 3 amino acids) as compared to wild type F1. Some amino acids may be deleted appropriately from the C terminus of F2 fragment of the original F protein. The number of amino acids to be deleted may be, for example, the same as the number of insertions, or may be selected from about 0 to 10 amino acids. The F protein may be prepared so that the N-terminus of F1 is directly linked downstream of the cleavage sequence, unless the linkage inhibits the process of protease cleavage and membrane fusion. Alternatively, the cleavage sequence may be linked with F 1 fragment *via* an appropriate spacer.

A desired vector system can be used as a vector for expressing the modified F protein. Plasmid vectors are preferably used. The vector may be transfected into cells at the time of virus production, or integrated beforehand into chromosomes in cells to establish transformed cells. A desired promoter for expression in mammalian cells can be used as a promoter to express the modified F protein. CA promoter is preferably used. A recombinase target sequence is preferably used to allow the induction of recombinant enzyme-specific expression (see Example 9).

More preferably, viruses produced in the present invention are preferably those belonging to Paramyxoviridae (including *Respirovirus, Rubulavirus,* and *Morbillivirus*) or derivatives thereof, and more preferably those belonging to the genus *Respirovirus* (also referred to as *Paramyxovirus)* or derivatives thereof. The derivatives include viruses that are genetically-modified so as not to impair their gene-transferring ability. Examples of viruses of the genus *Respirovirus* applicable to this invention are human parainfluenza virus-1 (HPIV-1), human parainfluenza virus-3 (HPIV-3), bovine parainfluenza virus-3 (BPIV-3), Sendai virus (also referred to as murine parainfluenza virus-1), and simian parainfluenza virus-10 (SPIV-10). A more preferred virus produced in this invention is the Sendai virus. Viruses may be derived from natural strains, wild strains, mutant strains, laboratory-passaged strains, artificially constructed strains, or the like.

In particular, SeV is known to be pathogenic in rodents causing pneumonia, but is not pathogenic for humans. This is also supported by a previous report that nasal administration of wild type SeV does not have severely harmful effects on non-human primates (Hurwitz, J.L. et al., Vaccine 15: 533-540, 1997; Bitzer, M. et al., J. Gene Med. 5: 543-553, 2003; Slobod, K.S. et al., Vaccine 22: 3182-3186, 2004). These SeV characteristics suggest that SeV vectors can be applied therapeutically for humans.

Genomic RNA of minus-strand RNA virus refers to RNA that has the function to form a ribonucleoprotein (RNP) with the viral proteins of a minus-strand RNA virus. Genes contained in the genome are expressed by the RNP, genomic RNA is replicated, and daughter RNPs are formed. In general, in the minus-strand RNA viral genome, viral genes are arranged as antisense sequences between the 3'-leader region and the 5'-trailer region. Between the ORFs of respective genes are a transcription ending sequence (E sequence) ― intervening sequence (I sequence) ― transcription starting sequence (S sequence), such that RNA encoding the ORF of each gene is transcribed as an individual cistron. Genomic RNAs comprise the antisense RNA sequences encoding N (nucleocapsid (also referred to as nucleoprotein))-, P (phospho)-, and L (large)-proteins, which are viral proteins essential for the expression of the group of genes encoded by an RNA, and for the autonomous replication of the RNA itself. The genomic RNAs may encode envelope-constituting proteins. However, defective vectors deficient in these genes can also be constructed.

Genes of Paramyxovirinae viruses are generally listed as follows. In general, N gene is also listed as "NP gene." HN that does not have a neuraminidase activity is listed as "H".

| | | | | | | | |
|---|---|---|---|---|---|---|---|
| *Respirovirus* | N | P/C/V | M | F | HN | - | L |
| *Rubulavirus* | N | P/V | M | F | HN | (SH) | L |
| *Morbillivirus* | N | P/C/V | M | F | H | - | L |

For example, the database accession numbers for the nucleotide sequences of each of the Sendai virus genes are: M29343, M30202, M30203, M30204, M51331, M55565, M69046, and X17218 for N gene; M30202, M30203, M30204, M55565, M69046, X00583, X17007, and X17008 for P gene; D11446, K02742, M30202, M30203, M30204, M69046, U31956, X00584, and X53056 for M gene; D00152, D11446, D17334, D17335, M30202, M30203, M30204, M69046, X00152, and X02131 for F gene; D26475, M12397, M30202, M30203, M30204, M69046, X00586, X02808, and X56131 for HN gene; and D00053, M30202, M30203, M30204, M69040, X00587, and X58886 for L gene. Examples of viral genes encoded by other viruses are: CDV, AF014953; DMV, X75961; HPIV-1, D01070; HPIV-2, M55320; HPIV-3, D10025; Mapuera, X85128; Mumps, D86172; MV, K01711; NDV, AF064091; PDPR, X74443; PDV, X75717; RPV, X68311; SeV, X00087; SV5, M81442; and Tupaia, AF079780 for N gene; CDV, X51869; DMV, Z47758; HPIV-1, M74081; HPIV-3, X04721; HPIV-4a, M55975; HPIV-4b, M55976; Mumps, D86173; MV, M89920; NDV, M20302; PDV, X75960; RPV, X68311; SeV, M30202; SV5, AF052755; and Tupaia, AF079780 for P gene; CDV, AF014953; DMV, Z47758; HPIV-1, M74081; HPIV-3, D00047; MV, AB016162; RPV, X68311; SeV, AB005796; and Tupaia, AF079780 for C gene; CDV, M12669; DMV, Z30087; HPIV-1, S38067; HPIV-2, M62734; HPIV-3, D00130; HPIV-4a, D10241; HPIV-4b, D10242; Mumps, D86171; MV, AB012948; NDV, AF089819; PDPR, Z47977; PDV, X75717; RPV, M34018; SeV, U31956; and SV5, M32248 for M gene; CDV, M21849; DMV, AJ224704; HPN-1, M22347; HPIV-2, M60182; HPIV-3, X05303; HPIV-4a, D49821; HPIV-4b, D49822; Mumps, D86169; MV, AB003178; NDV, AF048763; PDPR, Z37017; PDV, AJ224706; RPV, M21514; SeV, D17334; and SV5, AB021962 for F gene; and, CDV, AF112189; DMV, AJ224705; HPIV-1, U709498; HPIV-2, D000865; HPIV-3, AB012132; HPIV-4A, M34033; HPIV-4B, AB006954; Mumps, X99040; MV, K01711; NDV, AF204872; PDPR, Z81358; PDV, Z36979; RPV, AF132934; SeV, U06433; and SV-5, S76876 for HN (H or G) gene. However, multiple strains are known for each virus, and there are also genes that comprise sequences other than those cited above as a result of strain variation.

In general, a minus-strand RNA viral vector carrying N, P, and L genes autonomously expresses viral genes from the RNA genome in cells, and the genomic RNA is replicated. Furthermore, infectious viral particles are formed in the presence of envelope-constituting proteins and released to the outside of the cells. When the genome carries genes encoding envelope-constituting proteins required for the formation of infectious viral particles, the viral vector can propagate autonomously and become a transmissible viral vector. When any or all of the genes encoding envelope-constituting proteins are deleted from the genome, the viral vector is a deficient viral vector which cannot form infectious viral particles in infected cells.

The phrase "envelope-constituting proteins" refers to viral proteins which are components of the viral envelope, and include spike proteins exposed on the envelope surface, which function in infection or adhesion to cells, and lining proteins that function in envelope formation and such. Specifically, genes for envelope-constituting proteins include F (fusion), HN (hemagglutinin-neuraminidase)(or H (hemagglutinin)), and M (matrix). Some viral species have genes of H, M1, G, and such. Deficiency of the gene for F or HN (or H), which are spike proteins, is effective to convert the minus-strand RNA viral vector into a nontransmissible vector, while deficiency of the gene for M, which is an envelope lining protein, is effective to disable the particle formation in infected cells (WO 00/70055, WO 00/70070, and WO 03/025570; Li, H.-O. et al., J. Virol. 74(14) 6564-6569 (2000); Hasan, M. K. et al., 1997, J. General Virology 78: 2813-2820). Meanwhile, vectors deficient in any combination of at least two of F, HN (or H), and M genes assure greater safety. Genes can be deleted or made deficient by mutagenizing them for loss of protein function (WO 00/09700) or by removing the coding sequences. For example, minus-strand RNA virus deficient in both M and F genes (ΔMΔF) is a nontransmissible vector incapable of forming particles. The ΔMΔF virus retains strong infectivity and ability to express genes *in vitro* and *in vivo,* and the degrees are equivalent to those of the wild type virus. The ΔMΔF virus is expected to further contribute to the improvement of the safety of minus-strand RNA viral vectors. Viruses deficient in genes for envelope-constituting proteins can be prepared using mammalian cells that co-express the proteins complementing the deficiency as well as the modified F protein. In some cell types, infection does not require the HN protein (Markwell, M.A. et al., Proc. Natl. Acad. Sci. USA 82(4):978-982 (1985)), and is established with just the F protein. Viral vectors for introducing genes into such cells can be prepared by supplying only a modified F protein.

Further, when a defective virus is produced, a pseudotyped viral vector can be produced using an envelope protein different from that deleted from the viral genome. For example, when reconstituting a virus, a recombinant virus including a desired envelope protein can be generated by expressing an envelope protein other than the envelope protein originally encoded by the basic viral genome. Such proteins are not particularly limited. A desired protein that confers an ability to infect cells may be used. Examples of such proteins include envelope proteins of other viruses, for example, the G protein of vesicular stomatitis virus (VSV-G). The VSV-G protein may be derived from an arbitrary VSV strain. For example, VSV-G proteins derived from Indiana serotype strains (J. Virology 39: 519-528 (1981)) may be used, but the present invention is not limited thereto. Furthermore, the present vector may include any arbitrary combination of envelope proteins derived from other viruses. Preferred examples of such proteins are envelope proteins derived from viruses that infect human cells. Such proteins are not particularly limited, and include retroviral amphotropic envelope proteins and the like. For example, the envelope proteins derived from mouse leukemia virus (MuLV) 4070A strain can be used as the retroviral amphotropic envelope proteins. In addition, envelope proteins derived from MuMLV 10A1 strain may also be used (for example, pCL-10A1 (Imgenex) (Naviaux, R. K. et al., J. Virol. 70:5701-5705 (1996)). The proteins of *Herpesviridae* include, for example, gB, gD, gH, and gp85 proteins of herpes simplex viruses, and gp350 and gp220 proteins of EB virus. The proteins of *Hepadnaviridae* include the S protein of hepatitis B virus. These proteins may be used as fusion proteins in which the extracellular domain is linked to the intracellular domain of the F or HN protein. As described above, it is possible in this invention to produce pseudotype viral vectors that include envelope proteins, such as VSV-G, derived from viruses other than the virus from which the genome was derived. If the viral vectors are designed such that these envelope proteins are not encoded in RNA genomes, the proteins will never be expressed after virion infection of the cells.

A modified F protein with a modified protease cleavage sequence may have modifications at other sites in addition to the cleavage site. For example, the cell fusion ability can be potentiated by deleting the cytoplasmic domain of F protein (PCT/JP03/05528). For example, when an F protein made to lack some amino acids from the cytoplasmic domain so that the cytoplasmic domain has 0 to 28 amino acids, more preferably 1 to 27 amino acids, still more preferably 4 to 27 amino acids, the F protein has significantly strong cell-fusion ability as compared to the wild type F protein. The cytoplasmic domain refers to a domain located on the cytoplasmic side of a membrane protein The cytoplasmic domain of F protein corresponds to a C-terminal region of the transmembrane (TM) domain. For example, a vector having a stronger cell-fusion ability than a vector prepared using the wild type F protein can be obtained by preparing a viral vector carrying an F protein with a cytoplasmic domain of 6 to 20 amino acids, more preferably 10 to 16 amino acids, still more preferably 13 to 15 amino acids.

Alternatively, it is possible to construct viruses that have a fusion protein of two types of spike proteins. When the F protein, which is involved in cell fusion, and the HN protein (or H protein), which is thought to be involved in adhesion to cells, are expressed as a fusion protein, the virus has very strong cell-fusion ability as compared to when each protein is expressed separately (PCT/JP03/05528). This fusion protein is a protein in which the two proteins are linked at the respective cytoplasmic domains. Specifically, the fusion protein comprises the F protein on its N-terminal side and the HN (or H) protein on its C-terminal side. When the two proteins are fused, the full length proteins may be fused, or the HN (or H) protein is fused with an F protein lacking the whole cytoplasmic domain or a portion thereof.

Furthermore, the minus-strand RNA viral vector may be deficient in one or more accessory genes. For example, by knocking out the V gene, one of the SeV accessory genes, the pathogenicity of SeV toward hosts such as mice is remarkably reduced, without hindering gene expression and replication in cultured cells (Kato, A. et al., 1997, J. Virol. 71: 7266-7272; Kato, A. et al., 1997, EMBO J. 16: 578-587; Curran, J. et al., WO01/04272, EP1067179). Such attenuated vectors are particularly useful as nontoxic viral vectors for *in vivo* or *ex vivo* gene transfer.

Specifically, the methods for producing a minus-strand RNA viral vector comprise the steps of:
(a) transcribing the genomic RNA of minus-strand RNA virus or the complementary strand thereof in mammalian cells in the presence of:
   (i) a modified viral protein in which the protease cleavage sequence in F protein is modified into a cleavage sequence for an alternative protease,
   (ii) the alternative protease, and
   (iii) proteins constituting RNP comprising the genomic RNA of minus-strand RNA virus; and
(b) recovering the produced viruses.

The genomic RNA does not encode the modified viral protein. The genomic RNA of minus-strand RNA virus or the complementary strand thereof (antigenomic RNA) forms an RNP with the viral proteins constituting RNP of minus-strand RNA virus, expresses the viral proteins encoded by the genome, and amplifies the genome and antigenomic RNA s to form viral particles. The virus can be obtained by recovering the particles.

The "viral proteins constituting RNP" refer to a group of viral proteins that form a complex with genomic RNA of minus-strand RNA viruses and which are required for replication of the genomic RNA and expression of the genes encoded by the genome. Specifically, the proteins are: N (nucleocapsid (also referred to as nucleoprotein (NP))), P (phospho) and L (large) proteins. Although these notations vary in some viral species, the corresponding protein is obvious to those skilled in the art (Anjeanette Robert et al., Virology 247:1-6 (1998)). The viral RNP can be reconstituted by transcribing the minus strand, which is the same as the genome, or the plus strand (antigenome, which is the complementary strand of the genomic RNA). The plus strand is preferably generated to improve the efficiency of viral reconstitution. The RNA ends preferably match with the ends of the 3' leader sequence and the 5' trailer sequence of the natural viral genome as accurately as possible. This can be achieved by adding a self-cleaving ribozyme at the 5' end of the transcript to accurately cleave the end of the minus-strand RNA viral genome by the ribozyme (Inoue, K. et al. J. Virol. Methods 107, 2003, 229-236). In another embodiment, to accurately control the 5' end of the transcript, a bacteriophage RNA polymerase recognition sequence is used as the transcription start site and the RNA polymerase is expressed in cells. Such bacteriophage RNA polymerases include, for example, RNA polymerases of *E. coli* T3 and T7 phages, and *Salmonella* SP6 phage (Krieg, P.A. and Melton, D.A. 1987. In vitro RNA synthesis with SP6 RNA polymerase. Methods Enzymol. 155: 397-15; Milligan, J.F., Groebe, D.R., Witherell, G.W., and Uhlenbeck, O.C. 1987. Oligoribonucleotide synthesis using T7 RNA polymerase and synthetic DNA templates. Nucleic Acids Res. 15: 8783-798; Pokrovskaya, I.D. and Gurevich, V.V. 1994. In vitro transcription: Preparative RNA yields in analytical scale reactions. Anal. Biochem. 220: 420-23). The bacteriophage RNA polymerase may be expressed, for example, using vaccinia virus expressing the polymerase (Fuerst, T.R. et al., Proc. Natl. Acad. Sci. USA 83, 8122-8126(1986)) or non-viral vectors, such as plasmids (see Examples). It is also preferable to establish cell lines that can inducibly express a bacteriophage RNA polymerase by integrating a gene encoding the polymerase into chromosomes of virus-producing cells. It is possible to use the desired promoters for the expression in mammalian cells listed above. CA promoter is preferably used.

To regulate the 3'-end of the transcript, for example, a self-cleaving ribozyme can be encoded at the 3'-end of the transcript, allowing accurate cleavage of the 3'-end with this ribozyme (Hasan, M. K. et al., J. Gen. Virol. 78: 2813-2820, 1997; Kato, A. et al., 1997, EMBO J. 16: 578-587; and Yu, D. et al., 1997, Genes Cells 2: 457-466). An auto-cleaving ribozyme derived from the antigenomic strand of delta hepatitis virus can be used.

The viral genome can encode a desired foreign gene. A recombinant viral vector carrying a foreign gene can be obtained by inserting the foreign gene into the viral vector genome (Yu, D. et al., Genes Cells 2: 457-466, 1997; Hasan, M. K. et al., J. Gen. Virol. 78: 2813-2820, 1997). The foreign gene may be inserted into, for example, a desired position in non-coding regions of the viral genome, such as between the 3' leader region of the genomic RNA and the viral protein ORF closest to the 3' end, between each of the viral protein ORFs, and/or between the 5' trailer region and the viral protein ORF closest to the 5' end. Further, in genomes deficient in envelope proteins such as M, F, or HN gene, nucleic acids encoding foreign genes can be inserted into those deficient regions. When introducing a foreign gene into a paramyxovirus, it is desirable to insert the gene such that the chain length of the polynucleotide to be inserted into the genome will be a multiple of six (Kolakofski, D. et al., J. Virol. 72:891-899, 1998; Calain, P. and Roux, L., J. Virol. 67:4822-4830, 1993; Calain, P. and Roux, L., J. Virol. 67: 4822-4830, 1993). An E-I-S sequence should be arranged between the inserted foreign gene and the viral ORF (Tokusumi, T. et al. (2002) Virus Res 86(1-2), 33-8). Two or more foreign genes can be inserted in tandem via E-I-S sequences.

A cloning site for inserting a foreign gene can be designed in a genomic RNA -encoding cDNA so that the foreign gene can be readily inserted into the cDNA. The site may be placed, for example, at a desired position within the noncoding region of the genome. Specifically, such a gene can be inserted between the 3'-leader region and the viral protein ORF proximal to 3', between respective viral protein ORFs, and/or between the viral protein ORF proximal to 5' and the 5'-trailer region. When the genome lacks genes encoding an envelope-constituting protein, the cloning site can be designed to be in the region where the genes have been deleted. The cloning site may be, for example, a restriction enzyme recognition sequence.

Expression levels of a foreign gene carried in a vector can be controlled using the type of transcriptional initiation sequence added upstream (to the 3'-side of the minus strand (negative strand)) of the gene (WO01/18223). The expression levels can also be controlled by the position at which the foreign gene is inserted in the genome: the nearer to the 3'-end of the minus strand the insertion position is, the higher the expression level will be; and the nearer to the 5'-end the insertion position is, the lower the expression level will be. Thus, to obtain a desired gene expression level, the insertion position of a foreign gene can be appropriately controlled such that the combination with genes encoding the viral proteins before and after the foreign gene is most suitable.

For example, a desired S sequence of a minus-strand RNA virus may be used as the S sequence to be attached when inserting a foreign gene-encoding nucleic acid into the genome. The sequence 3'-UCCCWVUUWC-5' (W= A or C; V= A, C, or G)(SEQ ID NO: 23) can be preferably used for Sendai viruses. Particularly preferred sequences are 3'-UCCCAGUUUC-5' (SEQ ID NO: 24), 3'-UCCCACUUAC-5' (SEQ ID NO: 25), and 3'-UCCCACUUUC-5' (SEQ ID NO: 26). When shown as plus strand-encoding DNA sequences, these sequences are 5'-AGGGTCAAAG-3' (SEQ ID NO: 27), 5'-AGGGTGAATG-3' (SEQ ID NO: 28), and 5'-AGGGTGAAAG-3' (SEQ ID NO: 29). A preferred E sequence of a Sendai viral vector is, for example, 3'-AUUCUUUUU-5' (SEQ ID NO: 30) or 5'-TAAGAAAAA-3' (SEQ ID NO: 31) for the plus strand-encoding DNA. An I sequence may be, for example, any three nucleotides, specifically 3'-GAA-5' (5'-CTT-3' in the plus strand DNA).

Specific methods for producing minus-strand RNA viruses include, for example, methods for transiently producing viruses. One of such methods is the method of transfecting mammalian cells with a vector for transcribing DNA encoding a ribozyme and a genomic RNA of minus-strand RNA virus or the complementary strand thereof under the control of a mammalian promoter, and a vector for expressing viral proteins constituting RNP comprising the genomic RNA of minus-strand RNA virus. This method is carried out in the presence of a modified protein with a modified protease cleavage sequence, and the protease. Functional RNP is formed through the transcription of the genomic RNA of minus-strand RNA virus or the antigenomic RNA by a mammalian promoter in the presence of RNP constituting viral proteins. As a result viruses are reconstituted. The minus-strand RNA viral vector can be obtained by recovering the minus-strand RNA viruses produced in the cells or propagation products thereof.

In an alterative method, mammalian cells are transfected with a vector containing DNA encoding a bacteriophage RNA polymerase under the control of mammalian promoter, a vector containing DNA encoding a genomic RNA of minus-strand RNA virus or the complementary strand thereof linked downstream of the RNA polymerase recognition sequence, and a vector expressing viral proteins (N, L, and P) constituting RNP comprising the genomic RNA of minus-strand RNA virus. The transfection is carried out in the presence of a modified protein with a modified protease cleavage sequence and the protease. Functional RNP is formed through the transcription of the genomic RNA or antigenomic RNA of the minus-strand RNA virus by a mammalian promoter in the presence of RNP-constituting viral proteins. As a result viruses are reconstituted. The minus-strand RNA viral vector can be obtained by recovering the minus-strand RNA virus produced in the cells or propagation products thereof.

The vector used for transfection is preferably a plasmid. Each plasmid may be constructed to express a single protein. Alternatively, multiple proteins may be expressed on a single plasmid. For this purpose, a single plasmid may have multiple promoters. Alternatively, multiple proteins may be produced by a single promoter using IRES or such. The transfection-based viral production described above is superior because it can rapidly produce viruses without using special cells. Although a desired promoter for expression in mammalian cells can be used, the CA promoter is preferable.

A viral vector can also be used to express a bacteriophage RNA polymerase. For example, there is a known method for producing minus-strand RNA viruses using recombinant vaccinia virus vTF7-3, which expresses T7 RNA polymerase (Fuerst, T. R. et al., Proc. Natl. Acad. Sci. USA 83: 8122-8126,1986, Kato, A. et al., Genes Cells 1: 569-579, 1996), inactivated by irradiating ultraviolet light (UV) for 20 minutes (WO 00/70055, WO 00/70070, and WO 03/025570; Li, H.-O. et al., J. Virol. 74(14) 6564-6569 (2000); Hasan, M. K. et al., 1997, J. General Virology 78: 2813-2820). The vaccinia virus that may exist in the prepared minus-strand RNA virus solution can be completely removed by repeating amplification about three times with appropriately-diluted virus solutions.

In another embodiment of the method of the present invention, proteins and/or RNAs required for viral production are expressed from the chromosomes of virus-producing cells. One example of this method is a method using mammalian cell lines whose chromosomes contain integrated DNA which expresses a modified protein. Alternatively, this method also includes a method using mammalian cell lines whose chromosomes contain integrated DNA which becomes transcribed by a mammalian promoter into viral genomic RNA or the complementary strand thereof, or integrated DNA which is expressed as a bacteriophage-derived RNA polymerase by a mammalian promoter. Cells having the ability to produce higher titer viruses can be prepared by cloning transformants and selecting cells with high expression levels. Thus, such cells are useful for stably producing high titer viruses. In such cells, it is also preferred that the expression of viral genomic RNA and a protein of interest is designed to be responsive to and inducible upon stimulation, while in the absence of stimulation, neither is expressed by a mammalian promoter. Genes can be expressed inducibly by a mammalian promoter by using the loxP or FRT described above. The Cre recombinase or FLP recombinase is expressed at the time of viral production to induce the expression from a mammalian promoter. Although desired promoters for expression in mammalian cells can be used, a CA promoter is preferably used. A modified F protein gene can be introduced into a chromosome of cells that express viral genomic RNA and RNA polymerase.

In such cells, the minus-strand RNA virus can be reconstituted through transcription of viral genomic RNA or the complementary strand thereof and induction of the expression of modified F protein in the presence of viral proteins (N, L, and P) that constitute an RNP that contains the minus-strand RNA virus genomic RNA. RNP-constituting proteins may be supplied by transfecting plasmid vectors encoding the proteins.

In a method of excising the minus-strand RNA virus genome with a ribozyme (for example, the HamRbz method), the amount of each plasmid used in the transfection is for example: 0.1 to 2 µg (more preferably, 0.3 µg) of an NP-expressing plasmid; 0.1 to 2 µg (more preferably, 0.5 µg) of a P-expressing plasmid; 0.5 to 4.5 µg (more preferably, 2.0 µg) of an L-expressing plasmid; 0.1 to 5 µg (more preferably, 0.5 µg) of a modified-F-expressing plasmid; and 0.5 to 5 µg (more preferably, 5 µg) of a viral genomic RNA -encoding plasmid (plus or minus strand). To produce SeV, for example, the plasmids described in the Examples may be used in the following amounts:

| | |
|---|---|
| pCAGGS-NP | 0.1 to 2 µg (more preferably, 0.3 µg) |
| pCAGGS-P | 0.1 to 2 µg (more preferably, 0.5 µg) |
| pCAGGS-L(TDK) | 0.5 to 4.5 µg (more preferably, 2.0 µg) |
| pCAGGS-F5R | 0.1 to 5 µg (more preferably, 0.5 µg) |
| pCAGGS-SeV | 0.5 to 5 µg (more preferably, 5 µg) |
| (pCAGGS-SeV/ΔF-GFP) | |

In a method of transcribing the minus-strand RNA virus genome by a bacteriophage RNA polymerase, it is possible to use 0.1 to 2 µg (more preferably 0.5 µg) of an NP-expressing plasmid, 0.1 to 2 µg (more preferably 0.5 µg) of a P-expressing plasmid, 0.5 to 4.5 µg (more preferably 2.0 µg) of an L-expressing plasmid, 0.1 to 5 µg (more preferably 0.5 µg) of a modified-F-expressing plasmid, and 0.5 to 5 µg (more preferably 5 µg) of a viral genomic RNA -encoding plasmid (plus or minus strand). To produce SeV, for example, the plasmids described in the Examples can be used in the following amounts:

| | |
|---|---|
| pCAGGS-NP | 0.1 to 2 µg (more preferably, 0.5 µg) |
| pCAGGS-P | 0.1 to 2 µg (more preferably, 0.5 µg) |
| pCAGGS-L(TDK) | 0.5 to 4.5 µg (more preferably, 2.0 µg) |
| pCAGGS-F5R | 0.1 to 5 µg (more preferably, 0.5 µg) |
| pCAGGS-SeV | 0.5 to 5 µg (more preferably, 5 µg) |
| (pCAGGS-SeV/ΔF-GFP) | |

When envelope-constituting protein genes other than the F gene (for example, the HN gene and/or M gene) are also deleted from DNA encoding the viral genome, infectious viral particles are not formed. However, infectious virions can be formed by separately introducing host cells with these deleted genes, and/or genes encoding the envelope proteins of other viruses, and then expressing these genes therein (WO 00/70055 and WO 00/70070; Hirata, T. et al., 2002, J. Virol. Methods, 104:125-133; Inoue, M. et al., 2003, J. Virol. 77:6419-6429). When envelope-constituting proteins are expressed in virus-producing cells, a desired promoter for expression in mammalian cells can be used. CA promoter is preferably used.

After culturing for about 48 to 72 hours after transfection, cells are harvested, and then disintegrated by repeating freeze-thawing about three times. Cells are re-infected with the disintegrated materials including RNP, and cultured. Alternatively, the culture supernatant is recovered, added to a culture solution of cells to infect them, and the cells are then cultured. Transfection can be conducted by, for example, forming a complex with lipofectamine, polycationic liposome, or the like, and transducing the complex into cells. Specifically, various transfection reagents can be used. DOTMA (Roche), Superfect (QIAGEN #301305), DOTAP, DOPE, DOSPER (Roche #1811169), and TransIT-LT1 (Mirus, Product No. MIR 2300) may be given as examples. In order to prevent decomposition in the endosome, chloroquine may also be added (Calos, M. P., 1983, Proc. Natl. Acad. Sci. USA 80: 3015). In cells transduced with RNP, virus is amplified following progression of viral gene expression from RNP and RNP replication. Vectors thus recovered can be stored at -80°C. In order to reconstitute a nontransmissible virus lacking a gene encoding an envelope-constituting protein, cells expressing the envelope-constituting protein (helper cells) may be used for transfection, or a plasmid expressing the envelope-constituting protein may be cotransfected. Alternatively, an envelope-gene defective type virus can be amplified by further culturing the transfected virus-producing cells overlaid with cells expressing the envelope-constituting protein (see WO00/70055 and WO00/70070).

Known methods can be referred to for more details regarding the production of minus-strand RNA viruses(Hasan, M. K. et al., J. Gen. Virol. 78: 2813-2820, 1997; Kato, A. et al., 1997, EMBO J. 16: 578-587; and Yu, D. et al., 1997, Genes Cells 2: 457-466; WO97/16539; WO97/16538; WO00/70055; WO00/70070; WO03/025570; Durbin, A. P. et al., 1997, Virology 235: 323-332; Whelan, S. P. et al., 1995, Proc. Natl. Acad. Sci. USA 92: 8388-8392; Schnell. M. J. et al., 1994, EMBO J. 13: 4195-4203; Radecke, F. et al., 1995, EMBO J. 14: 5773-5784; Lawson, N. D. et al., Proc. Natl. Acad. Sci. USA 92: 4477-4481; Garcin, D. et al., 1995, EMBO J. 14: 6087-6094; Kato, A. et al., 1996, Genes Cells 1: 569-579; Baron, M. D. and Barrett, T., 1997, J. Virol. 71: 1265-1271; Bridgen, A. and Elliott, R. M., 1996, Proc. Natl. Acad. Sci. USA 93: 15400-15404). When the method of the present invention is applied to these methods, minus strand RNA viruses including parainfluenza virus, vesicular stomatitis virus, rabies virus, measles virus, rinderpest virus, and Sendai virus can be reconstituted from DNA with high efficiency.

Titers of viruses thus recovered can be determined, for example, by measuring CIU (Cell Infecting Unit) or hemagglutination activity (HA) (WO00/70070; Kato, A. et al., 1996, Genes Cells 1: 569-579; Yonemitsu, Y. & Kaneda, Y., Hemaggulutinating virus of Japan-liposome-mediated gene delivery to vascular cells. Ed. by Baker AH. Molecular Biology of Vascular Diseases. Method in Molecular Medicine: Humana Press: pp. 295-306, 1999). Titers of vectors carrying GFP (green fluorescent protein) marker genes and the like can be quantified by directly counting infected cells, using the marker as an indicator (for example, as GFP-CIU). Titers thus measured can be treated in the same way as CIU (WO00/70070).

So long as a virus can be reconstituted, the host cells used in the reconstitution are not particularly limited. For example, in the reconstitution of Sendai virus vectors and the like, cultured cells such as LLC-MK2 cells and CV-1 cells derived from monkey kidney, BHK cells derived from hamster kidney, and cells derived from humans can be used. Especially, the methods of the present invention can produce viruses using human cells, which has been conventionally difficult. Further, to obtain a large quantity of a Sendai virus vector, a viral vector obtained from an above-described host can be used to infect embrionated hen eggs to amplify the vector. Methods for manufacturing viral vectors using hen eggs have already been developed (Nakanishi, et al., ed. (1993), "State-of-the-Art Technology Protocol in Neuroscience Research III, Molecular Neuron Physiology", Koseisha, Osaka, pp. 153-172). Specifically, for example, a fertilized egg is placed in an incubator, and cultured for nine to twelve days at 37 to 38°C to grow an embryo. After the viral vector is inoculated into the allantoic cavity, the egg is cultured for several days (for example, three days) to proliferate the viral vector. Conditions such as the period of culture may vary depending upon the recombinant Sendai virus being used. Then, allantoic fluids including the vector are recovered. Separation and purification of a Sendai virus vector from allantoic fluids can be performed according to a usual method (Tashiro, M., "Virus Experiment Protocol," Nagai, Ishihama, ed., Medical View Co., Ltd., pp. 68-73, (1995)).

According to the method for producing viruses as described herein, the minus strand RNA viral vector can be released into extracellular fluid of virus producing cells at a titer of, for example, 1 x 10⁵ CIU/ml or higher, preferably 1 x 10⁶ CIU/ml or higher, more preferably 5 x 10⁶ CIU/ml or higher, more preferably 1 x 10⁷ CIU/ml or higher, more preferably 5 x 10⁷ CIU/ml or higher, more preferably 1 x 10⁸ CIU/ml or higher, and more preferably 5 x 10⁸ CIU/ml or higher. The titer of virus can be determined according to methods described herein or elsewhere (Kiyotani, K. et al., Virology 177(1), 65-74 (1990); and WO00/70070).

The viral vectors produced by the method of the present invention can be purified to be substantial pure. The purification can be achieved using known purification/separation methods, including filtration, centrifugation, adsorption, and column purification, or any combinations thereof. The phrase "substantially pure" means that the virus component constitutes a major proportion of a solution of the viral vector. For example, a viral vector composition can be confirmed to be substantially pure by the fact that the proportion of protein contained as the viral vector component to the total protein (excluding proteins added as carriers and stabilizers) in the solution is 10% (w/w) or greater, preferably 20% or greater, more preferably 50% or greater, preferably 70% or greater, more preferably 80% or greater, and even more preferably 90% or greater. Specific purification methods for, for example, the paramyxovirus vector includes methods using cellulose sulfate ester or cross-linked polysaccharide sulfate ester (Japanese Patent Application Kokoku Publication No. (JP-B) S62-30752 (examined, approved Japanese patent application published for opposition), JP-B S62-33879, and JP-B S62-30753) and methods including adsorbing to fucose sulfate-containing polysaccharide and/or degradation products thereof (WO97/32010), but are not limited thereto.

In the production of compositions containing the viral vector of the present invention, the vector may be combined with desired pharmaceutically acceptable carriers or media according to needs. The "pharmaceutically acceptable carriers or media" refers to materials that can be administered together with the vector and that do not significantly inhibit the gene transfer via the vector. Such carriers and media include, for example, sterile water, sodium chloride solution, dextrose solution, lactated Ringer's solution, culture medium, serum, and phosphate buffered saline (PBS). They may be appropriately combined with the vector to formulate a composition. The composition of the present invention may also include membrane stabilizers for liposome (for example, sterols such as cholesterol). The composition may also include antioxidants (for example, tocopherol or vitamin E). In addition, the composition may also include vegetable oils, suspending agents, detergents, stabilizers, biocidal agents, and the like. Furthermore, preservatives and other additives may also be added. The formula of the present composition may be aqueous solution, capsule, suspension, syrup, or the like. The composition of the present invention may also be in a form of solution, freeze-dried product, or aerosol. When it is a freeze-dried product, it may include sorbitol, sucrose, amino acids, various proteins, and the like as a stabilizer. The viral vector compositions thus produced are useful as reagents and pharmaceuticals for gene introduction into cells of desired mammals and animals, including humans and non-human mammals. Particularly, viral vectors produced by the method of the present invention are suitably used in gene therapy for humans.

### Examples

Hereinafter, the present invention will be explained in more detail with reference to the Examples, but is not to be construed as being limited thereto. All the references cited herein are incorporated as parts of the present specification.

### [Example 1] Insertion of a furin recognition sequence into an F gene-expressing plasmid

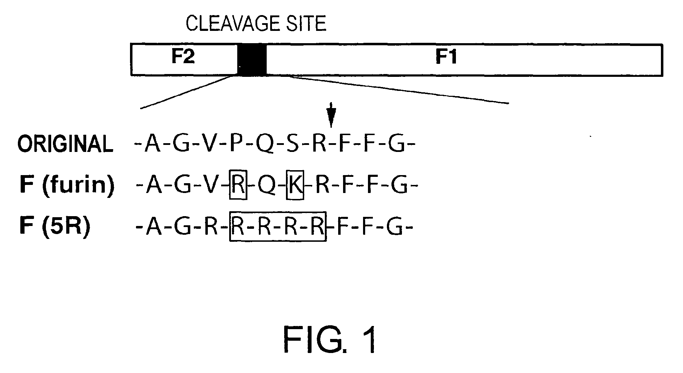

A furin recognition sequence was introduced into the F protein to be expressed in packaging cells to create packaging cells (packaging cells based on human cells, in particular) that allow recovery of an F gene-deficient SeV vector (SeV/ΔF) using cells other than LLC-MK2. The insertion sequences were designed based on R-(X)-R/K-R, a consensus sequence recognized by furin (Chambers, T.J. et al., Annu. Rev. Microbiol. 44, 649-688 (1990)). Two types of sequences: R-Q-K-R (F (furin) sequence), which is the least modified sequence as compared with the sequence of the original F protein, and (R)-R-R-R-R (F(5R) sequence), which is expected to be cleaved more efficiently, were used (Fig. 1). Furthermore, F gene was expressed using a Cre/loxP expression induction system. To construct the system, the plasmid pCALNdLw, which was designed to inducibly express gene products by Cre DNA recombinase (Arai, T. et al., J. Virol. 72: 1115-1121 (1988)), was used as in the case of establishing the LLC-MK2-based packaging cells for the F protein (LLC-MK2/F7 cell) (Li, H.-O. et al., J. Virology 74, 6564-6569 (2000), WO 00/70070).

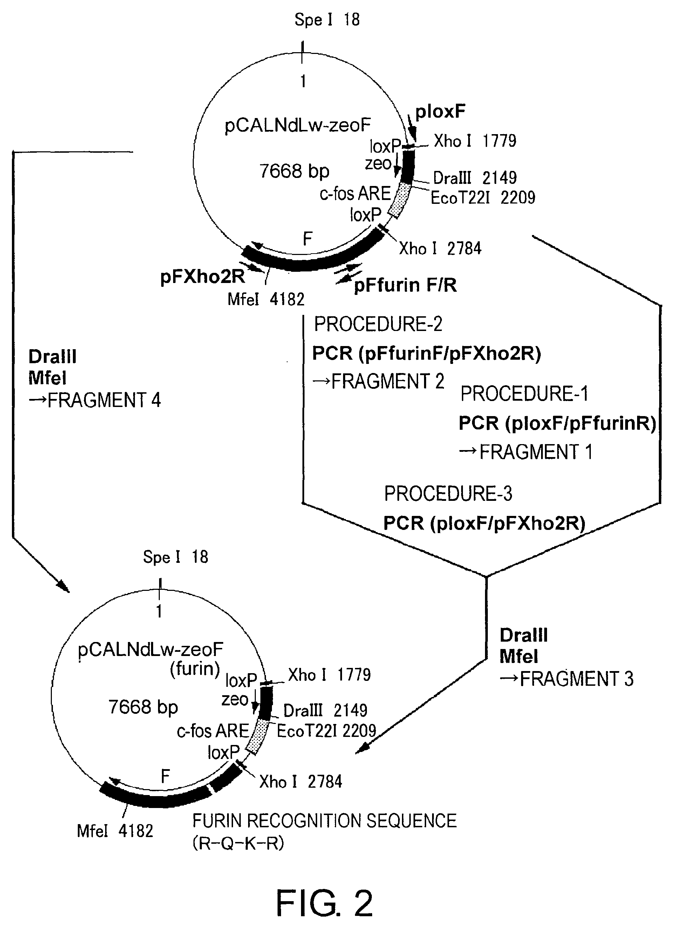

First, the furin recognition sequence (R-Q-K-R: F(furin)) was inserted into the F gene using a plasmid (pCALNdLw-ZeoF: Japanese Patent Application No. 2001-283451) constructed by inserting the F gene into pCALNdLw carrying Zeocin resistance gene. An outline of the subcloning procedure is shown in Fig. 2. PCR was carried out using Pfu Turbo (STRATAGENE, La Jolla, CA), pCALNdLw-ZeoF as a template, and a pair of the following primers: ploxF (5'-CATTTTGGCAAAGAATTGATTAATTCGAG-3'/SEQ ID NO: 32) and pFfurinR (5'-TCACAGCACCCAAGAATCTCTTCTGGCGAGCACCGGCATTTTGTGTC-3'/SEQ ID NO: 33) (procedure 1). Likewise, PCR was carried out using pCALNdLw-ZeoF as a template, and a pair of the following primers: pFfurinF (5'-GACACAAAATGCCGGTGCTCGCCAGAAGAGATTCTTGGGTGCTGTGA-3'/SEQ ID NO: 34) and pFXho2R (5'- GATCGTAATCACAGTCTCTCGAGAGTTGTACCATCTACCTAC -3'/SEQ ID NO: 35) (procedure 2). Each of the primers pFfurinR and pFfurinF used in the PCR has a sequence for mutagenizing the furin recognition sequence (R-Q-K-R in this case). After separating the PCR products by agarose gel electrophoresis, a band of 1470 bp obtained by procedure 1 and a band of 1190 bp obtained by procedure 2 were each excised and recovered using GENE CLEAN KIT (Funakoshi, Tokyo) (referred to as fragments (i) and (ii), respectively). 1 µl each of the purified, 10-times-diluted fragments (i) and (ii) was combined together. PCR was further carried out using Pfu Turbo and a pair of primers ploxF and pXho2R (procedure 3). 5 µl of the PCR product was electrophoresed in an agarose gel, and stained with ethidium bromide. As a result, a band of 2.6 kbp was detected as expected. Then, the remaining PCR product was purified using Qiaquick PCR Extraction kit (QIAGEN, Bothell, WA). The product was then sequentially digested with the restriction enzymes *Dra*III and *Mfe*I. After separation by agarose gel electrophoresis, a band of about 2.0 kbp was excised (fragment 3). Separately, pCALNdLw-Zeo-F was digested sequentially with *Dra*III and *Mfe*I and then separated by agarose gel electrophoresis. A band of about 6 kbp was excised, and the DNA was purified with GENE CLEAN KIT (fragment 4). These fragments 3 and 4 were ligated together to construct pCALNdLw-Zeo-F(furin) (also referred to as pCALNdLw-Zeo-F furin).

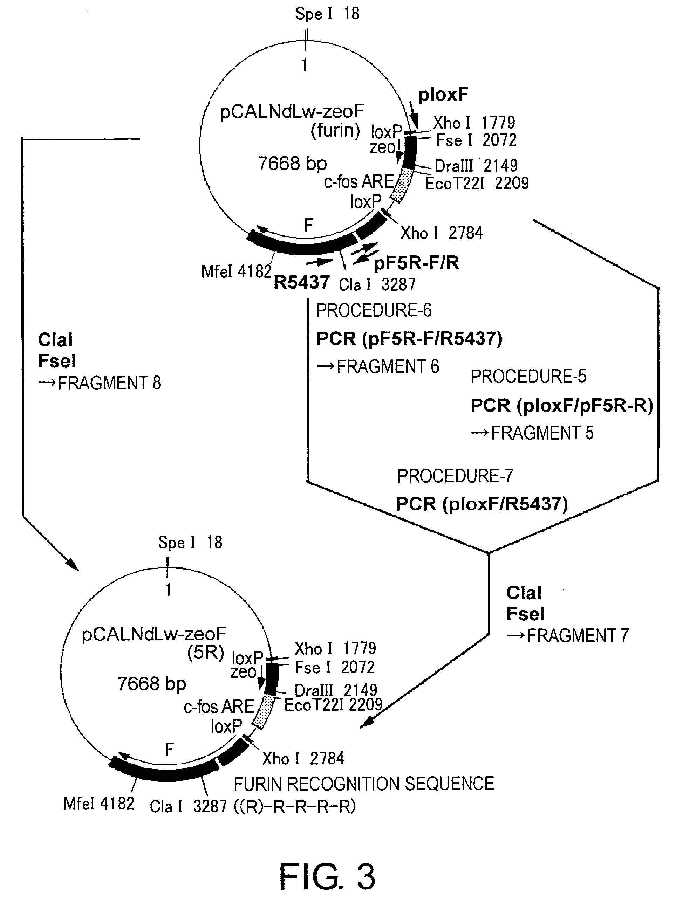

Next, the more efficiently-cleaved furin recognition sequence ((R)-R-R-R-R:F(5R)) was introduced into the F gene in pCALNdLw-ZeoF. An outline of the subcloning procedure is shown in Fig. 3. PCR was carried out using as a template pCALNdLw-Zeo-F(furin) prepared as described above and a pair of primers ploxF and pF5R-R (5'-TCACAGCACCGAAGAATCTCCTCCGGCGACGACCGGCATTTTGTGTCGTATC-3'/SEQ ID NO: 36) (procedure 5). Likewise, PCR was also carried out using pCALNdLw-Zeo-F(furin) as a template and a pair of primers pF5R-F (5'-GATACGACACAAAATGCCGGTCGTCGCCGGAGGAGATTCTTCGGTGCTGTGA-3'/SEQ ID NO: 37) and R5437(5'-AAATCCTGGAGTGTCTTTAGAGC-3'/SEQ ID NO: 38) (procedure 6). The primers pF5R-R and pF5R-F used in the PCR each have a sequence for mutagenizing the furin recognition sequence ((R)-R-R-R-R in this case). After the PCR product was separated by agarose gel electrophoresis, a band of 1470 bp obtained by procedure 5 and a band of 200 bp obtained by procedure 6 were each excised and purified using Qiaquick Gel Extraction kit (referred to as fragments 5 and 6, respectively). 1 µl each of the purified, 50-times-diluted fragments 5 and 6 was combined together, and PCR was further carried out using Pfu Turbo and a pair of primers ploxF and R5437 (procedure 7). After 5 µl of the PCR product was separated by agarose gel electrophoresis and stained, a band of about 1.6 kbp was detected. Then, the remaining was purified with Qiaquick PCR Purification kit, and then digested with *Cla*I and *Fse*I. After separation by agarose gel electrophoresis, a band of about 1 kbp was excised and purified with Qiaquick PCR Purification kit (fragment 7). Separately, pCALNdLw-Zeo-F(furin) was digested with *Cla*I and *Fse*I, and separated by agarose gel electrophoresis. A band of about 8 kbp was then excised and purified with Qiaquick PCR Purification kit (fragment 8). These fragments 7 and 8 were ligated together to construct pCALNdLw-Zeo-F(5R) (also referred to as pCALNdLw-Zeo F5R). This pCALNdLw-Zeo F5R was digested with *Xho*I. After purification and ligation, DNA containing no *Xho*I fragment (including Zeocin resistance gene) was selected to obtain pCAGGS-F5R.

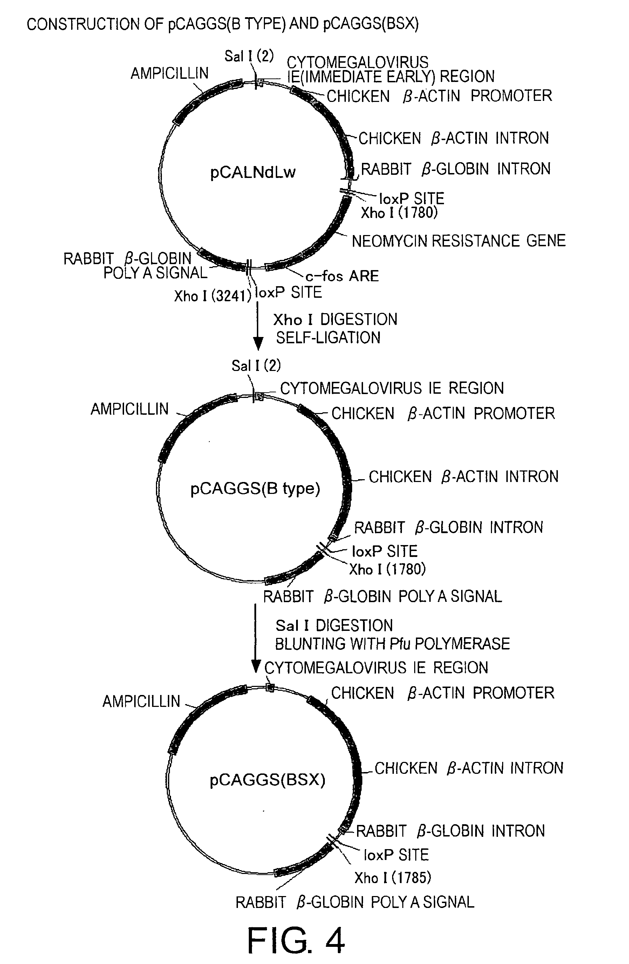

### [Example 2] Construction of other plasmids (Fig. 4)

### Construction of pCAGGS (B type)

pCALNdLw (Arai, T. et al. J. Virology 72, 1998, p.1115-1121) was digested with *Xho*I, purified using the Qiaquick PCR Purification kit, and ligated. The plasmid from which the *Xho*I fragment was deleted was selected and named pCAGGS (B type). pCAGGS (B type) was digested with *Sal*I, and blunted using the Pfu DNA polymerase. The DNA was purified with the Qiaquick PCR Purification kit, and ligated. The plasmid in which the *Sal*I site was deleted was selected and named pCAGGS(BSX)

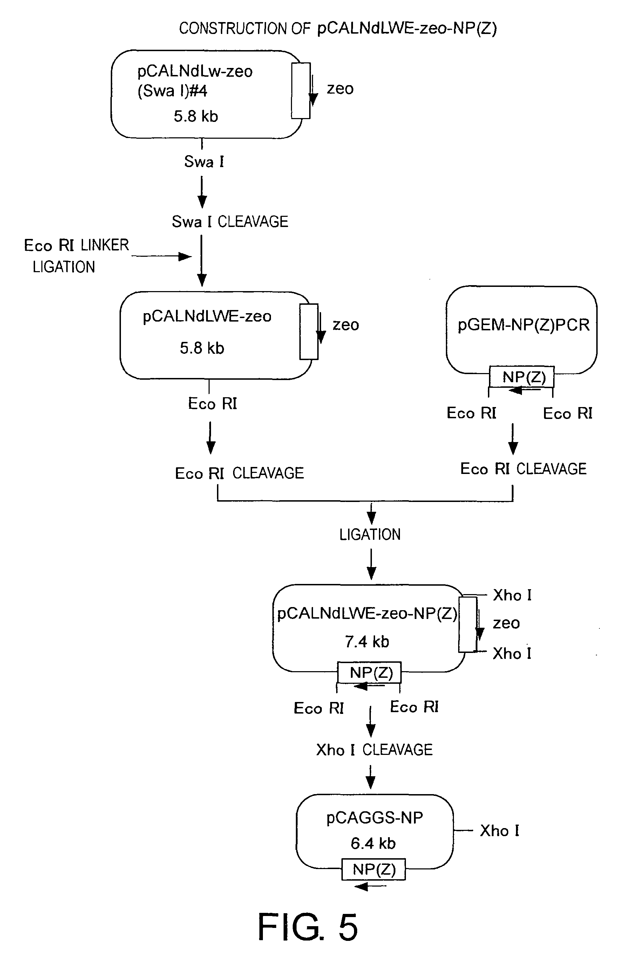

### Construction of pCAGGS-NP (Fig. 5)

pCALNdLw was digested with *Spe*I and *Eco*T22I, and separated by agarose gel electrophoresis. The 2651-bp and 3674-bp fragments were excised, and purified with the Qiaquick gel Extraction kit. The 2651-bp fragment was then digested with *Xho*I*.* After separation by agarose gel electrophoresis, a 1760-bp band was purified. The Zeocin resistance gene was amplified by PCR using pcDNA3.1/Zeo(+) as template and the following primers:
5'-TCTCGAGTCGCTCGGTACGATGGCCAAGTTGACCAGTGCCGTTCCGGTGCTCAC-3' (SEQ ID NO: 39) and
5'-AATGCATGATCAGTAAATTACAATGAACATCGAACCCCAGAGTCCCGCTCAGTCCT GCTCCTCGGCCACGAAGTGCACGCAGTTG-3' (SEQ ID NO: 40). The amplified DNA was digested with *Xho*I and *Eco*T22I, and separated by agarose gel electrophoresis. A band of 438 bp was excised and purified using the Qiaquick Gel Extraction kit. The three fragments, namely the fragment comprising the Zeocin resistance gene and the 3674-bp and 1761-bp fragments described above, were ligated to each other to yield pCALNdLw-Zeo. This pCALNdLw-Zeo was digested with *Swa*I and an *Eco*RI linker (STRATAGENE) was inserted to yield pCALNdLWE-Zeo. A Sendai virus cDNA introduced with a multi-cloning site (JP-A 2002-272465) (hereinafter referred to as pSeV(TDK)) was digested with *Not*I and *Xho*I, and separated by agarose gel electrophoresis. A band of 1669 bp was excised and purified using the Qiaquick Gel Extraction kit. The fragment comprising the NP gene was inserted into pGEM11Zf(+) (Promega), which had been digested with *Not*I and *Xho*I, to yield pGEM-NP(Z)PCR14-3. PCR amplification was performed using this plasmid as template and the following primers: 5'-CCGGAATTCAACAAATGGCCGGGTTGTTGAGCACCTTCGA-3' (SEQ ID NO: 41) and 5'-CCGGAATTCCTAGATTCCTCCTATCCCAGCTACTGCTGCTCG-3' (SEQ ID NO: 42). The PCR product was digested with *Eco*RI, and inserted into the *Eco*RI site of pCALNdLWE-Zeo to yield pCALNdLWE-Zeo-NP(Z). Then, pCALNdLWE-Zeo-NP(Z) was digested with *Xho*I, and ligated to construct a plasmid from which the *Xho*I fragment was deleted. The resulting plasmid was named pCAGGS-NP.

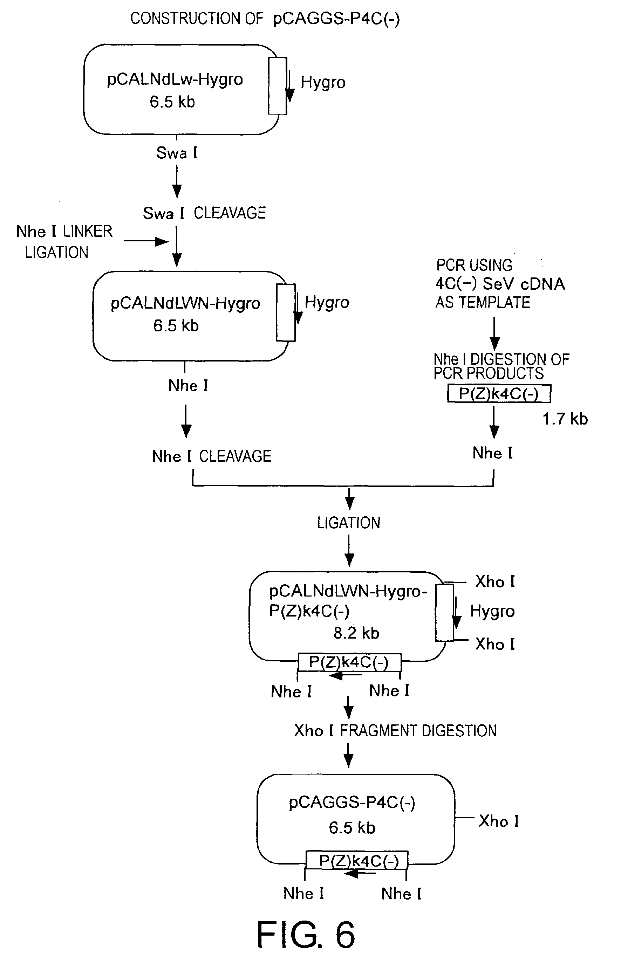

### Construction of pCAGGS-P4C(-) (Fig. 6)

pCALNdLw-HygroM (Inoue, M. et al. J. Virology 77, 2003, p6419-6429) was digested with *Xho*I*,* and separated by agarose gel electrophoresis. A 1679-bp band containing the hygromycin resistance gene was excised and purified using the Qiaquick Gel Extraction kit. pCALNdLw was digested with *Xho*I*.* After agarose gel electrophoresis, a 4864-bp band was excised and purified using the Qiaquick Gel Extraction kit. These two fragments were ligated to each other to construct pCALNdLw-Hygro. This pCALNdLw-Hygro was digested with *Swa*I*,* and an *Nhe*I linker (STRATAGENE) was inserted to yield pCALNdLWN-Hygro. PCR was carried out with the KOD-PLUS DNA Polymerase (ToYoBo), using 4C(-)SeV cDNA (Kurotani, Kato, Nagai, et al Genes to Cells 3, 1998, p111-124) as template and the following primers: 5'-CTAGCTAGCCCACCATGGATCAAGATGCCTTCATTCTAAAAGAAGATTCT-3' (SEQ ID NO: 43) and 5'-CTAGCTAGCCTAGTTGGTCAGTGACTCTATGTCCTCTTCTACGAGTTCCA-3' (SEQ ID NO: 44). The PCR product was purified using the Gene Clean kit, and then digested with *Nhe*I. The product was purified with the Gene Clean kit. This was inserted into the *Nhe*I site of pCALNdLWN-hygro described above to yield pCALNdLWN-hygro-P(Z)k4C(-). This plasmid was digested with *Xho*I, and then purified with the Qiaquick PCR Purification kit. After ligation, the plasmid from which the *Xho*I fragment (hygromycin resistance gene region) was deleted was selected to yield pCAGGS-P4C(-).

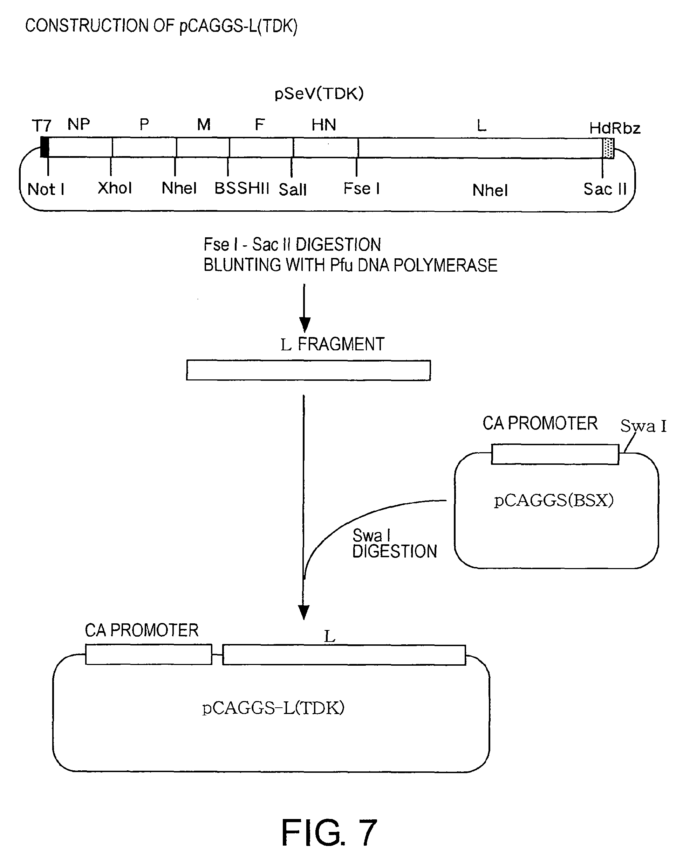

### Construction of pCAGGS-L(TDK) (Fig. 7)

pSeV(TDK) was digested with *Fse*I and *Sac*II and separated by agarose gel electrophoresis. A 6732-bp band was excised and purified using the Qiaquick Gel Extraction kit. The fragment was blunted by reacting with the Pfu DNA Polymerase and dNTP at 72°C for 10 minutes. After purification with the Qiaquick PCR Purification kit, the fragment was inserted into the *Swa*I site of pCAGGS(BSX) to yield pCAGGS-L(TDK).

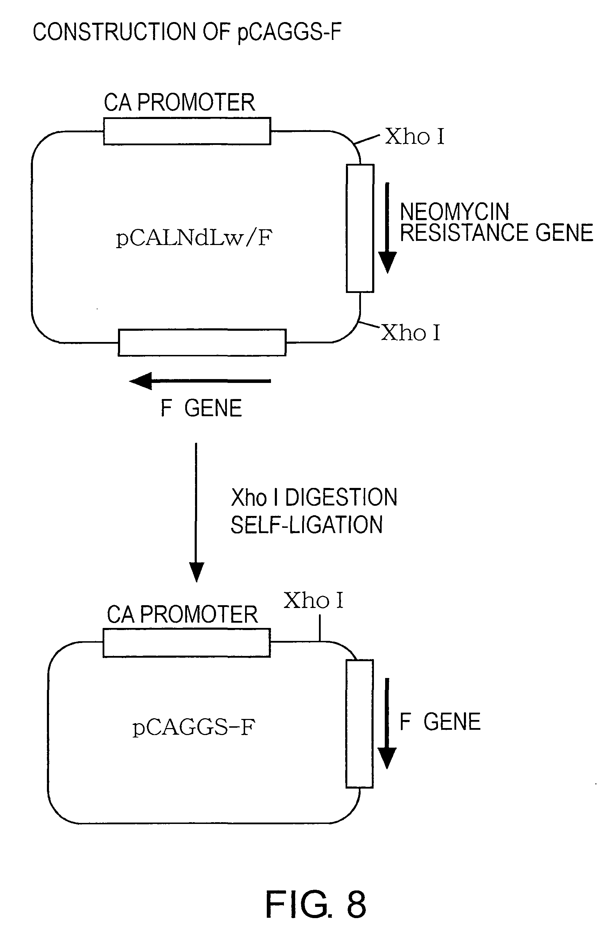

### Construction of pCAGGS-F (Fig. 8)

pCALNdLw/F (Li, H.-O. et al. J. Virology 74, 2000, p6564-6569) was digested with *Xho*I*.* After purification, the plasmid was ligated. The plasmid from which the *Xho*I fragment (Neomycin resistance gene region) was deleted was selected to obtain pCAGGS-F.

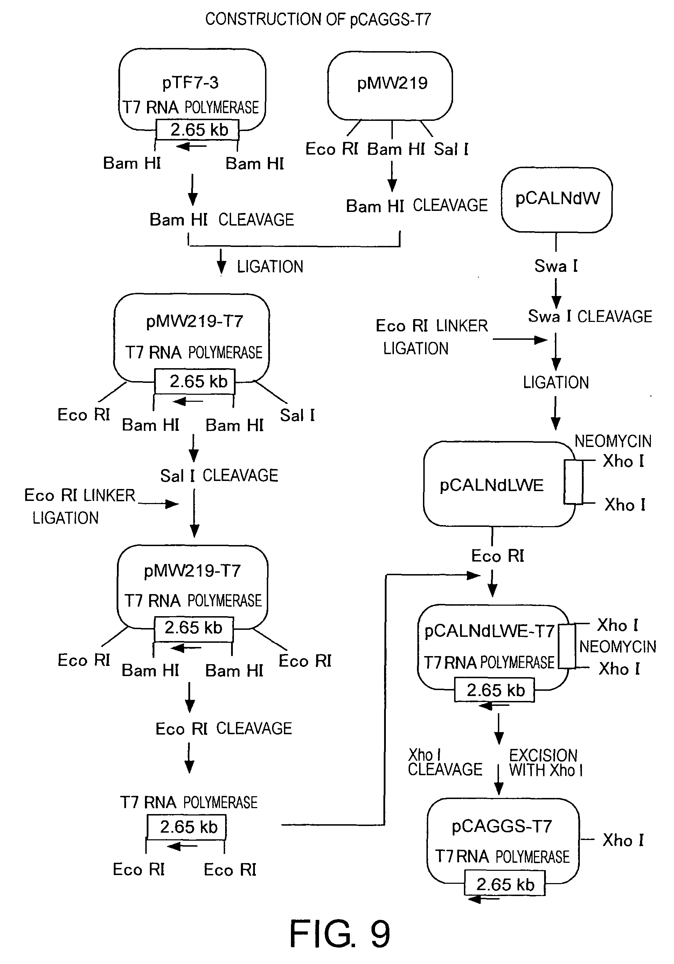

### Construction of pCAGGS-T7 (Fig. 9)

pTF7-3 (ATCC No.67202) was digested with *Bam*HI*.* After separation by agarose gel electrophoresis, a fragment of 2.65 kbp comprising the T7 RNA polymerase gene was recovered and inserted into the *Bam*HI site of pMW219 (Nippon Gene Co. Ltd.) to yield pMW219-T7. This pMW219-T7 was digested with *Sal*I and blunted using the DNA Blunting kit (TaKaRa). An *Eco*RI linker (Stratagene #901026) was inserted to yield pMW219-T7-Eco RI. This pMW219-T7-Eco RI was digested with *Eco*RI. An *Eco*RI fragment comprising the T7 RNA polymerase was purified and inserted into the *Eco*RI site of pCALNdLWE described above to yield pCALNdLWE-T7.

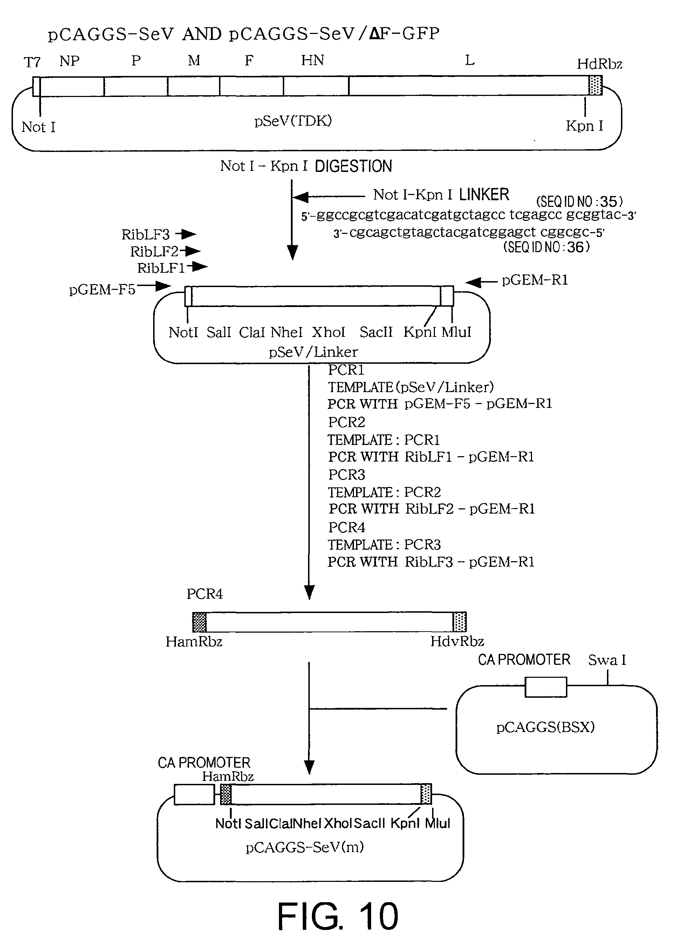

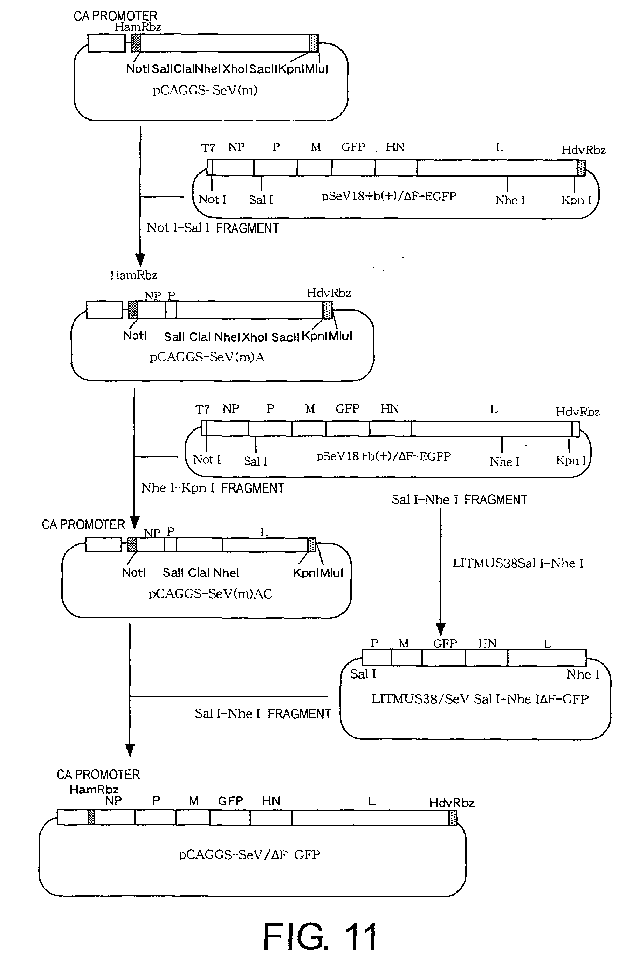

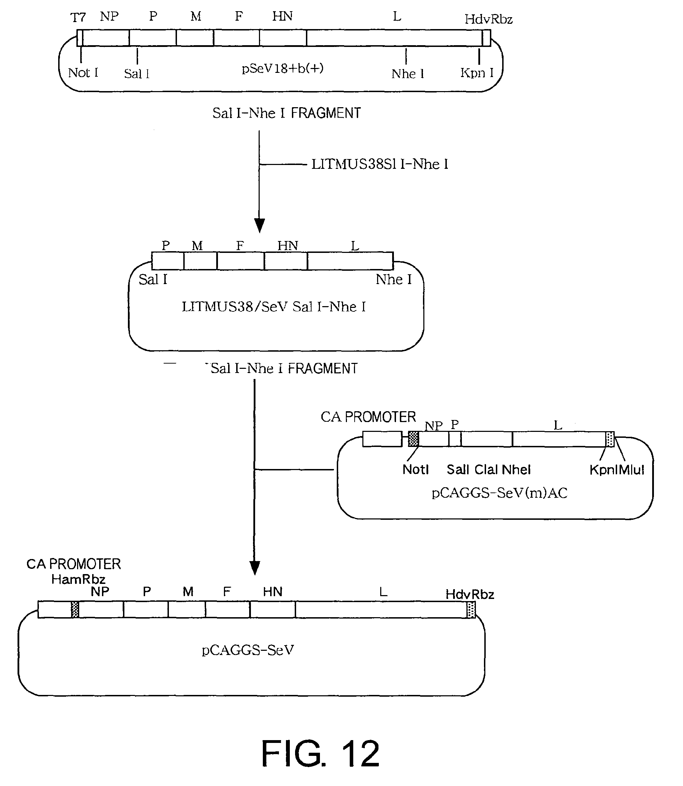

### Construction of pCAGGS-SeV and pCAGGS-SeV/ΔF-GFP (Figs. 10 to 12)

pSeV(TDK) was digested with *Not*I and *Kpn*I and separated by agarose gel electrophoresis. A band of 2995 bp was then excised and purified using the Qiaquick Gel Extraction kit. 2 µg (2 µl) each of MlinkerF:
5'-GGCCGCGTCGACATCGATGCTAGCCTCGAGCCGCGGTAC-3' (SEQ ID NO: 45) and MlinkerR: 5'-CGCGGCTCGAGGCTAGCATCGATGTCGACGC-3' (SEQ ID NO: 46) was mixed with 21 µl of H₂O and annealed at 95°C for 5 minutes, 85°C for 15 minutes, 65°C for 15 minutes, 37°C for 15 minutes, 25°C for 15 minutes, and then 4°C. The mixture and a solution of purified pSeV(TDK) *Not*I*-Kpn*I were ligated to yield pSeV/Linker. PCR was carried out using the pSeV/Linker as template, KOD-Plus (TOYOBO), and the following primers: pGEM-F5: 5'-CTTAACTATGCGGCATCAGAGC-3' (SEQ ID NO: 47) and pGEM-R1:
5'-GCCGATTCATTAATGCAGCTGG-3' (SEQ ID NO: 48). The PCR product was purified using the Qiaquick PCR Purification kit. PCR was carried out using a solution of the purified PCR product as template, KOD-PLUS (TOYOBO), and the following primers: RibLF1:
5'-CTATAGGAAAGGAATTCCTATAGTCACCAAACAAGAG-3' (SEQ ID NO: 49) and pGEM-R1: 5'-GCCGATTCATTAATGCAGCTGG-3' (SEQ ID NO: 48). The PCR product was purified using the Qiaquick PCR Purification kit. PCR was carried out using a solution of the purified PCR product as template, KOD-PLUS (TOYOBO), and the following primers: RibLF2:
5'-GATGAGTCCGTGAGGACGAAACTATAGGAAAGGAATTC-3' (SEQ ID NO: 50) and pGEM-R1: 5'-GCCGATTCATTAATGCAGCTGG-3' (SEQ ID NO: 48). The PCR product was purified using the Qiaquick PCR Purification kit. Furthermore, PCR was carried out using a solution of the purified PCR product as template, KOD-PLUS (TOYOBO), and the following primers: RibLF3: 5'-GCGGGCCCTCTCTTGTTTGGTCTGATGAGTCCGTGAGGAC-3' (SEQ ID NO: 51) and pGEM-R1; 5'-GCCGATTCATTAATGCAGCTGG-3' (SEQ ID NO: 48). The PCR product was purified using the Qiaquick PCR Purification kit. This purified PCR product was inserted into the *Swa*I site of pCAGGS(BSX) to yield pCAGGS-SeV(m). Then, pseV18+b(+)/ΔF-EGFP (Li, H.-O. et al. J. Virology 74, 2000, p6564-6569) was digested with *Not*I and *Sal*I, and separated by agarose gel electrophoresis. A band of 1972 bp was excised, purified using the Qiaquick Gel Extraction kit, and digested with *Not*I and *Sal*I. The resulting fragment was ligated with purified pCAGGS-SeV(m) to yield pCAGGS-SeV(m)A. pSeV(+) 18/ΔF was digested with *Nhe*I and *Kpn*I and separated by agarose gel electrophoresis. A band of 3325 bp was excised, purified using the Qiaquick Gel Extraction kit, and digested with *Not*I and *Sal*I. The resulting fragment was ligated with pCAGGS-SeV(m) to yield pCAGGS-SeV(m)AC.

pSeV18+b(+) (Li, H.-O. et al. J. Virology 74, 2000, p6564-6569) was digested with *Sal*I and *Nhe*I, and purified with the Qiaquick PCR purification kit. The resulting fragment was inserted into the *Sal*I*-Nhe*I site of LITMUS38 (NEW ENGLAND BioLabs) to yield Litmus38/SeV Sal I-Nhe I. This Litmus38/SeV Sal I-Nhe I was digested with *Sal*I and *Nhe*I and separated by agarose gel electrophoresis. A band of 9886 bp was excised and purified using the Qiaquick Gel Extraction kit. The DNA was inserted into the *Sal*I*-Nhe*I site of pCAGGS-SeV(m)AC to yield pCAGGS-SeV.

pSeV/ΔF-EGFP (Li, H.-O. et al. J. Virology 74, 2000, p6564-6569) was digested with *Sal*I and *Nhe*I. The resulting fragment was purified with the Qiaquick PCR purification kit, and inserted into the *Sal*I*-Nhe*I site of LITMUS38 (NEW ENGLAND BioLabs) to yield Litmus38/Sal I-Nhe IΔF-GFP. This Litmus38/Sal I-Nhe IΔF-GFP was digested with *Sal*I and *Nhe*I and separated by agarose gel electrophoresis. A band of 8392 bp was then excised and purified using the Qiaquick Gel Extraction kit. The DNA was inserted into the *Sal*I*-Nhe*I site of pCAGGS-SeV(m)AC to yield pCAGGS-SeV/ΔF-GFP.

### Construction of pGEM-IRES-Luci

A luciferase fragment, which was obtained by digesting pMAMneo-Luci(Clontech) with *Bam*HI, was inserted into the *Bam*HI site of pTM1 (Nature, 348, 1, November, 1990, 91-92) to construct pGEM-IRES-Luci.

### [Example 3] Establishment of T7 RNA Polymerase-expressing BHK-21 (hereinafter referred to as BHK/T7)

Using a mammalian transfection kit (Stratagene) or SuperFect (Qiagen), BHK-21 cells were transfected with pCALNdLWE-T7 that was constructed as described above. The cells were cultured for 2 weeks in D-MEM containing 400 µg/ml G418 at 37°C under 5% CO₂, yielding drug-resistant clones grown from a single cell. The resulting drug-resistant clones were infected with recombinant adenovirus (AxCANCre) that expresses Cre DNA recombinase at an Moi of 4. After 24 hours, the cells were washed once with PBS and harvested. The expression of T7 RNA polymerase was confirmed by Western blotting analysis using a rabbit polyclonal anti-T7 RNA polymerase antibody.

Clones that were confirmed to express the T7 RNA polymerase were transfected with pGEM-IRES-Luci using SuperFect. The cells were harvested after 24 hours, and their luciferase activity was measured with MiniLumat LB9506 (EG&G BERTHOLD) using the Dual Luciferase Reporter System (Promega) kit to confirm the activity of the T7 RNA polymerase.

### [Example 4] Recovery of F-deficient SeV vector

F gene-deficient SeV was recovered by a method using a vector that transcribes a Sendai virus genome attached to a hammerhead ribozyme (hereinafter referred to as the HamRbz method). Human embryonic kidney-derived 293T cells were plated into 6-well plates at 1 x 10⁶ cells/well in 2 ml of 10% FBS-containing D-MEM. The transfection was carried out by the procedure described below. 30 µl of Opti-MEM was combined with 15 µl of TransIT-LT1 (Mirus), and incubated at room temperature for 10 to 15 minutes. During the incubation, a DNA solution was prepared. 0.3 µg of pCAGGS-NP, 0.5 µg of pCAGGS-P4C(-), 2 µg of pCAGGS-L(TDK), 0.5 µg of pCAGGS-F5R, and 0.5 to 5 µg of pCAGGS-SeV/ΔF-GFP were dissolved in 20 µl of Opti-MEM. After 10 to 15 minutes, the DNA solution was combined with the TransIT-LT1 solution and allowed to stand at room temperature for 15 minutes. During this period, the cell culture medium was removed, and fresh 10% FBS-containing D-MEM was gently added at 1 ml/well. After 15 minutes, 500 µl of Opti-MEM (GIBCO) was added to the DNA-TransIT-LT1 mixture. The whole mixture was added to cells and cultured. After culturing for 72 hours at 37°C under 5% CO₂, the medium was discarded. The LLC-MK2/F7/A cells (cells in which the F protein expression is induced are referred to as "LLC-MK2/F7/A"; Li, H.-O. et al., J. Virology 74. 6564-6569 (2000); WO 00/70070) were suspended at 1 x 10⁶ cells/ml in (serum-free) MEM containing 7.5 µg/ml trypsin (hereinafter referred to as "Try-MEM") and the suspension was overlaid at 1 ml/well. The cells were cultured at 37°C under 5% CO₂. After 24 hours, 1 ml of the culture medium was collected and 1 ml of fresh Try-MEM was added. The cells were cultured at 37°C under 5% CO₂. After 48 hours, 1 ml of the culture medium was collected and 1 ml of fresh Try-MEM was added. The cells were cultured at 37°C under 5% CO₂. After 72 hours, 1 ml of the culture medium was collected. 133 µl of 7.5% BSA (final concentration: 1% BSA) was added to the collected culture media, and stored at -80°C prior to CIU measurement.

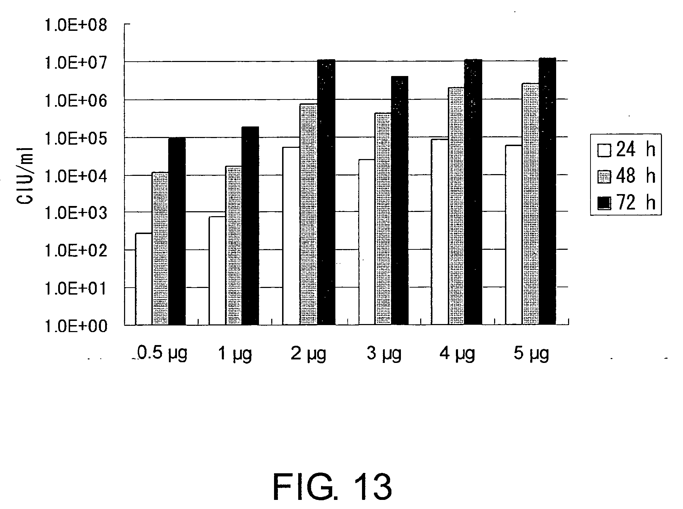

CIU was determined by counting GFP-expressing cells (GFP-CIU) as follows. 2 to 3 days before a CIU assay, LLC-MK2 cells were plated into 12-well plates. When plated 2 days before the assay, the cells were plated at a cell density of 1.5 x 10⁵ cells/well in 10% FBS-containing MEM (1 ml/well). When plated 3 days before the assay, the cells were plated at a cell density of 8.0 x 10⁴ cells/well in 10% FBS-containing MEM (1 ml/well). On the day of CIU assay, the cells were washed once with serum-free MEM. Ten-fold serial dilutions of the culture medium collected 24, 48, and 72 hours after overlaying the cells were prepared using MEM. After one hour of infection at 37°C, the cells were washed once with MEM and 1 ml of MEM was added thereto. After 2 days of culture at 37°C, the cells were observed under a fluorescence microscope to count GFP-positive cells in appropriately diluted wells. As a result, 1 x 10⁵ to 1 x 10⁷ GFP-CIU/ml of the viral vector was found to be recovered 72 hours after overlaying the cells (Fig. 13).

### [Example 5] Comparison of productivity by supplying F protein introduced with a furin recognition sequence (hereinafter referred to as "F5R"), as compared with wild-type F protein (hereinafter referred to as "F")]

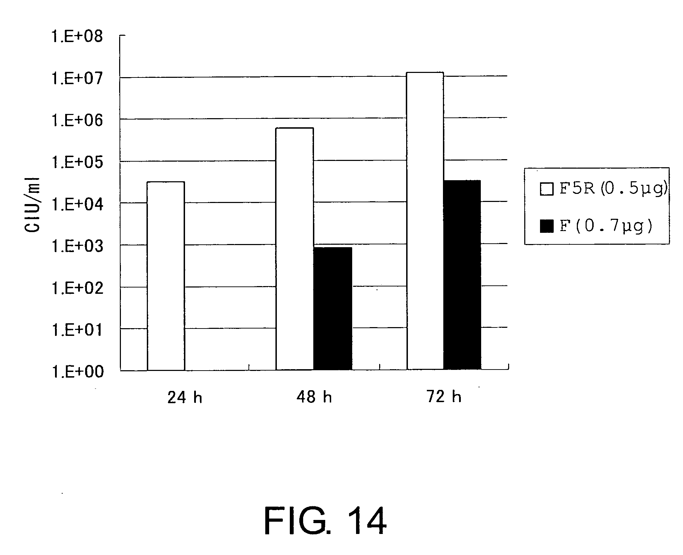

The reconstitution efficiency of supplying the F protein using pCAGGS was compared between the wild-type F gene and furin recognition sequence-introduced F5R. 293T cells were plated onto 6-well plates at 1 x 10⁶ cells/well in 2 ml of 10% FBS-containing D-MEM the day before transfection. The transfection was carried out by the procedure described below. 15 µl of TransIT-LT1 (Mirus) was mixed with 30 µl of Opti-MEM, and incubated at room temperature for 10 to 15 minutes. A DNA solution was prepared during the incubation. Fixed amounts of pCAGGS-NP (0.3 µg), pCAGGS-P4C(-) (0.5 µg), pCAGGS-L (2 µg), and pCAGGS-SeV/ΔF-GFP (2 µg), and various amounts of pCAGGS-F or pCAGGS-F5R (0.1, 0.3, 0.5, 0.7, and 0.9 µg) were dissolved in 20 µl of Opti-MEM. After 10 to 15 minutes, a DNA solution was mixed with the TransIT-LT1 solution and allowed to stand at room temperature for 15 minutes. During this period, the cell culture medium was removed, and fresh 10% FBS-containing D-MEM was gently added at 1 ml/well. After 15 minutes, 500 µl of Opti-MEM (GIBCO) was added to the DNA-TransIT-LT1 mixture. The whole mixture was added to cells and cultured. After culturing at 37°C under 5% CO₂ for 72 hours, the culture medium was discarded, and the LLC-MK2/F7/A cells were suspended at 1 x 10⁶ cells/ml in (serum-free) MEM containing 7.5 µg/ml trypsin (hereinafter referred to as "Try-MEM") and the suspension was overlaid at 1 ml/well. The cells were cultured at 37°C under 5% CO₂. After 24 hours, 1 ml of the culture medium was collected, and 1 ml of fresh Try-MEM was added. The cells were cultured at 37°C under 5% CO₂. After 48 hours, 1 ml of the culture medium was collected and 1 ml of fresh Try-MEM was added. The cells were cultured at 37°C under 5% CO₂. After 72 hours, 1 ml of the culture medium was collected. 133 µl of 7.5% BSA (final concentration: 1% BSA) was added to the collected culture medium, and stored at -80°C prior to CIU measurement. CIU assays were carried out after all samples were collected. As a result, when pCAGGS-F was used, the reconstitution efficiency was found to be the highest at 0.7 µg. The samples collected 24, 48, and 72 hours following the cell overlay contained 0, 7.9 x 10², and 3.3 x 10⁴ CIU/ml of viral vectors, respectively. Meanwhile, when pCAGGS-F5R was used, the reconstitution efficiency was found to be the highest at 0.5 µg. The samples collected 24, 48, and 72 hours following the cell overlay contained 3.2 x 10⁴, 5.7 x 10⁵, and 1.2 x 10⁷ CIU/ml of viral vectors, respectively. A comparison of the two in conditions of the highest reconstitution efficiency, pCAGGS-F5R gave a much higher reconstitution efficiency than pCAGGS-F, and yielded 373 times more viral vectors at 72 hours after the cell overlay (Fig. 14).

### [Example 6] Production of transmissible Sev vectors

Transmissible SeV vectors were recovered by a method for producing viral vectors in which a SeV genome is transcribed by T7 RNA polymerase using pCAGGS-T7 (hereinafter referred to as the pCAGGS-T7 method)

### 5-1 [Recovery of transmissible SeV vectors]

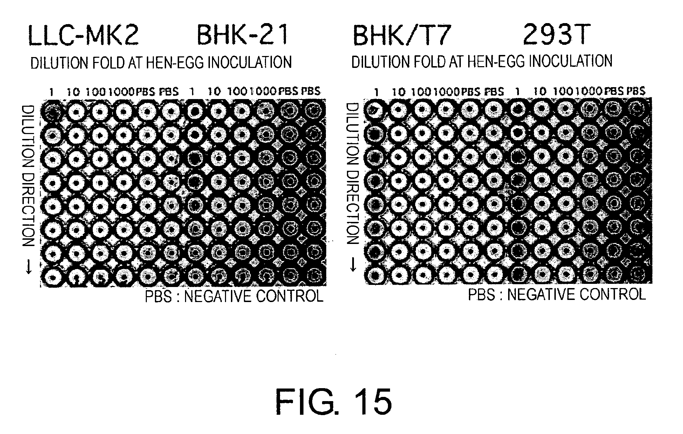

The day before transfection, each cell line was plated into 6-well plates (293T cell: 1 x 10⁶ cells/well/2 ml 10% FBS-containing D-MEM; LLC-MK2 cell: 5.0 x 10⁵ cells/well in 2 ml of 10% FBS-containing D-MEM; BHK-21 cell: 2.5 x 10⁵ cells/well in 2 ml of 10% FBS-containing D-MEM; BHK/T7 cell: 2.5 x 10⁵ cells/well in 2 ml of 10% FBS-containing D-MEM). The transfection was carried out by the procedure described below. 30 µl of Opti-MEM was mixed with 15 µl of TransIT-LT1 (Mirus) and incubated at room temperature for 10 to 15 minutes. A DNA solution was prepared during the incubation. 0.5 µg of pCAGGS-T7, 0.5 µg of pCAGGS-NP, 0.5 µg of pCAGGS-P4C(-), 2 µg of pCAGGS-L(TDK), and 5 µg of pSeV(TDK)18+GFP were dissolved in 20 µl of Opti-MEM. After 10 to 15 minutes, the DNA solution was mixed with the TransIT-LT1 solution and allowed to stand at room temperature for 15 minutes. During this period, the cell culture medium was removed, and fresh 10% FBS-containing D-MEM was gently added at 1 ml/well. After 15 minutes, 500 µl of Opti-MEM (GIBCO) was added to the DNA-TransIT LT1 mixture. After the whole mixture was added to cells, they were cultured at 37°C under 5% CO₂ for 3 days. Then, GFP-positive cells were counted, and the results were: 293T: 246 cells; LLC-MK2: 16 cells; BHK-21: 288 cells; and BHK/T7: 405 cells. The culture medium was then discarded, and 1 ml of PBS(-) was added to the cells. The cells were scraped using a cell scraper and collected in Eppendorf tubes. After freeze-thawing once, 100 µl of undiluted cell suspensions and cell suspensions diluted 10, 100, and 1000 times with PBS(-) were inoculated into 10-day hen eggs. The eggs were incubated in an incubator at 35°C for 3 days. Then, the chorioallantoic fluids were collected and analyzed by an HA assay. As a result, viral propagation was detected in the hen eggs inoculated with undiluted suspensions of 293T cells, BHK-21 cells, and BHK/T7 cells (Fig. 15).

### [Example 7] Production of F-deficient SeV vectors (II)

293T cells were plated into 6-well plates at 1 x 10⁶ cells/well in 2 ml of 10% FBS-containing D-MEM the day before transfection. The transfection was carried out by the procedure described below. 30 µl of Opti-MEM was combined with 15 µl of TransIT-LT1 (Mirus), and incubated at room temperature for 10 to 15 minutes. A DNA solution was prepared during the incubation. 0.5 µg of pCAGGS-T7, 0.5 µg of pCAGGS-NP, 0.5 µg of pCAGGS-P4C(-), 2 µg of pCAGGS-L(TDK), 0.5 µg of pCAGGS-F5R, and 0.5 to 5 µg of pSeV/ΔF-GFP (WO 00/70070) were dissolved in 20 µl of Opti-MEM. After 10 to 15 minutes, the DNA solution was combined with the TransIT LT1 solution and allowed to stand at room temperature for 15 minutes. During this period, the cell culture medium was removed, and fresh 10% FBS-containing D-MEM was gently added at 1 ml/well. After 15 minutes, 500 µl of Opti-MEM (GIBCO) was added to the DNA-TransIT-LT1 mixture. The whole mixture was added to the cells and cultured. After culturing at 37°C under 5% CO₂ for 72 hours, the culture medium was discarded, and the LLC-MK2/F7/A cells were suspended at 1 x 10⁶ cells/ml in Try-MEM and the suspension was overlaid at 1 ml/well. The cells were cultured at 37°C under 5% CO₂. After 24 hours, 1 ml of the culture medium was collected, and 1 ml of fresh Try-MEM was added. The cells were cultured at 37°C under 5% CO₂. After 48 hours, 1 ml of the culture medium was collected, and 1 ml of fresh Try-MEM was added. The cells were cultured at 37°C under 5% CO₂. After 72 hours, 1 ml of the culture medium was collected. 133 µl of 7.5% BSA (final concentration: 1% BSA) was added to the collected culture medium, and stored at -80°C prior to CIU measurement.

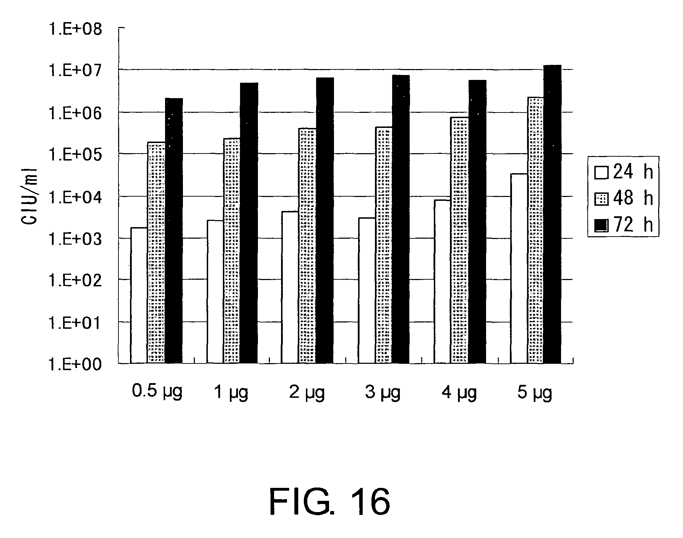

CIU was determined by counting GFP-expressing cells (GFP-CIU) as follows. 2 to 3 days before a CIU assay, the LLC-MK2 cells were plated onto 12-well plates. When plated 2 days before the assay, the cells were plated at 1.5 x 10⁵ cells/well in 1 ml/well of 10% FBS-containing MEM. When plated 3 days before the assay, the cells were plated at 8.0 x 10⁴ cells/well in 1 ml/well of 10% FBS-containing MEM. On the day of CIU assay, the cells were washed once with serum-free MEM. Ten-fold serial dilutions of the culture media collected 24, 48, and 72 hours after the cell overlay were prepared using MEM. After one hour of infection at 37°C, the cells were washed once with MEM and 1ml of MEM was added thereto. After 2 days of culture at 37°C, the cells were observed under a fluorescence microscope to count GFP-positive cells in adequately diluted wells. As a result, 1 x 10⁶ to 1 x 10⁷ GFP-CIU/ml of the viral vector was found to be recovered 72 hours after the cell overlay (Fig. 16).

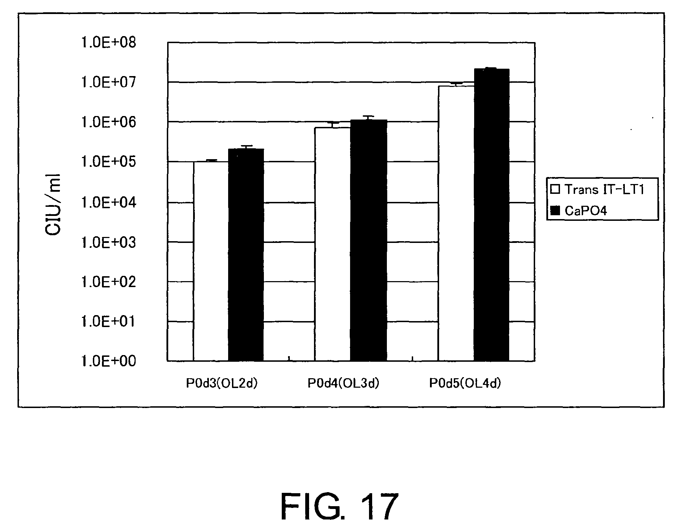

In the pCAGGS-T7 method, SeV18+ GFP/ΔF was also successfully recovered by the calcium phosphate method when 293T cells are used for introduction of the plasmid. The efficiency was comparable to or higher than that of TransIT-LT1 (Fig. 17).

### [Example 8] Evaluation of cell types used for the pCAGGS-T7 method

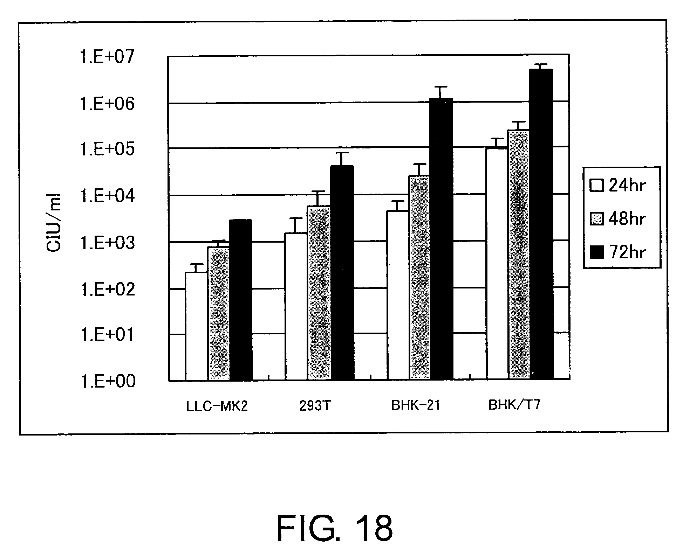

To assess whether the pCAGGS-T7 method can recover Sendai virus vectors by using cell lines other than 293T, it was tested whether the vector could be harvested using the LLC-MK2, BHK-21, BHK/T7, or 293T cell line. The day before transfection, each cell line was plated onto 6-well plates (LLC-MK: 5 x 10⁵ cells/well; BHK-21: 2.5 x 10⁵ cells/well; BHK/T7: 2.5 x 10⁵ cells/well; 293T: 1.0 x 10⁶ cells/well). The transfection was carried out by the procedure described below. 30 µl of Opti-MEM was mixed with 15 µl of TransIT-LT1 (Mirus), and incubated at room temperature for 10 to 15 minutes. A DNA solution was prepared during the incubation. 0.5 µg of pCAGGS-T7, 0.5 µg of pCAGGS-NP, 0.5 µg of pCAGGS-P4C(-), 2 µg of pCAGGS-L(TDK), 0.5 µg of pCAGGS-FSR, and 2 µg of pSeV/ΔF-GFP were dissolved in 20 µl of Opti-MEM (however, pCAGGS-T7 was not added when BHK/T7 cells were used). After 10 to 15 minutes, the DNA solution was mixed with the TransIT-LT1 solution and allowed to stand at room temperature for 15 minutes. During this incubation, the cell culture medium was removed, and fresh 10% FBS-containing D-MEM was gently added at 1 ml/well. After 15 minutes, 500 µl of Opti-MEM (GIBCO) was added to the DNA-TransIT-LT1 mixture. The whole mixture was added to cells and cultured. After culturing at 37°C under 5% CO₂ for 72 hours, the culture medium was discarded, and the LLC-MK2/F7/A cells were suspended at 1 x 10⁶ cells/ml in Try-MEM and the suspension was overlaid at 1 ml/well. The cells were cultured at 37°C under 5% CO₂. After 24 hours, 1 ml of the culture medium was collected, and 1 ml of fresh Try-MEM added to the cells. The cells were cultured at 37°C under 5% CO₂. After 48 hours, 1 ml of the culture medium was collected, and 1 ml of fresh Try-MEM was added to the cells. The cells were cultured at 37°C under 5% CO₂. After 72 hours, 1 ml of the culture medium was collected. 133 µl of 7.5% BSA (final concentration: 1% BSA) was added to the collected culture medium, and stored at -80°C prior to CIU measurement. It was found that the vector could be recovered with all the cell types tested (n=3). The vector recovery rate was in the order of high to low: BHK/T7 cell >BHK-21 cell >293T cell >LLC-MK2 cell (Fig. 18). Since BHK/T7 was not transfected with pCAGGS-T7, it was demonstrated that F-deficient SeV/ΔF-GFP could also be recovered using a CA promoter in T7-expressing cell lines.

### [Example 9] Cloning of packaging cells inducibly expressing F protein comprising a furin recognition sequence

Packaging cells were created using HeLa cell, a human cell line. Transfection was carried out using LipofectAMINE PLUS reagent (Invitrogen, Groningen, Netherlands) according to the method described in the protocol. Specifically, the method used is as follows: HeLa cells were plated in 6-well plates at 2 x 10⁵ cells/dish and cultured in D-MEM containing 10% FBS for 24 hours. 1.5 µg of pCALNdLw-Zeo-F(furin) or pCALNdLw-Zeo-F(5R) was diluted with D-MEM free of FBS and antibiotics (in a total volume of 94 µl). After stirring, 6 µl of LipofectAMINE PLUS reagent was added. The resulting mixture was stirred and allowed to stand at room temperature for 15 minutes. Then, LipofectAMINE reagent (4 µl of LipofectAMINE pre-diluted with D-MEM free of FBS and antibiotics to a total volume of 100 µl) was added and the resulting mixture was allowed to stand at room temperature for 15 minutes. Then, 1 ml of FBS-, antibiotic-free D-MEM was added to the mixture. After stirring, the transfection mixture was added to the HeLa cells washed once with PBS. After the cells were cultured at 37°C in a 5% CO₂ incubator for 3 hours, 1 ml of D-MEM containing 20% FBS was added thereto without removing the transfection mixture. The cells were cultured for 24 hours, and then detached by trypsin. The cells were diluted to about 5 cells/well or 25 cells/well in 96-well plates, and cultured in D-MEM containing 10% FBS and 500 µg/ml Zeocin (Gibco-BRL, Rockville, MD) for about two weeks. Clones grown from single cells were expanded to 6-well plate cultures. The clones prepared as described above were analyzed.

The F protein expression level in each obtained clone was semi-quantitatively analyzed by Western-blotting. Each clone was cultured almost until confluent in 6-well plates, and the cells were infected with Cre DNA recombinase-expressing recombinant adenovirus (AxCANCre) diluted with MEM containing 5% FBS at a MOI of 5 using the method of Saito *et al.* (Saito, I. et al., Nucl. Acid. Res. 23, 3816-3821 (1995); Arai, T. et al., J. Virol. 72, 1115-1121 (1998)). After two days of culture at 32°C, the culture supernatants were discarded and the cells were washed once with PBS. The cells were detached with a cell scraper and harvested. After performing SDS-PAGE by applying 1/10 volume of this sample to each lane, Western-blotting was carried out using an anti-F antibody (y236: Segawa, H. et al., J. Biochem. 123, 1064-1072 (1998)) to semi-quantitatively analyze F protein in cells. The Western-blotting was carried out by the following method. Cells harvested from a single well of 6-well plates were frozen at -80°C, and then lysed with 100 µl of 1x diluted SDS-PAGE sample buffer (Red Loading Buffer Pack; New England Biolabs, Beverly, MA). The lysates were heated at 98°C for 10 minutes. After centrifugation, 10 µl of the supernatants were loaded onto an SDS-PAGE gel (Multigel 10/20; Daiichi Pure Chemicals Co., Ltd, Tokyo, Japan). After electrophoresis at 15 mA for 2.5 hours, the proteins were transferred onto PVDF membrane (Immobilon PVDF transfer membrane; Millipore, Bedford, MA) at 100 mA for one hour by the semi-dry method. The transfer membrane was left in a blocking solution (Block Ace; Snow Brand Milk Products Co., Ltd, Sapporo, Japan) at 4°C for one hour or more. The membrane was soaked in a primary antibody solution containing 10% Block Ace and 1/1000 volume of an anti-F antibody at 4°C overnight. After the membrane was washed three times with TBS containing 0.05% Tween20 (TBST) and then three times with TBS, it was soaked in a secondary antibody solution containing 10% Block Ace and 1/5000 volume of HRP-conjugated anti-mouse IgG+IgM antibody (Goat F(ab')2 Anti-Mouse IgG+IgM, HRP; BioSource Int., Camarillo, CA) at room temperature for one hour while shaking. The membrane was washed three times with TBST and then three times with TBS. The detection was then carried out by the chemiluminescence method (ECL western blotting detection reagents; Amersham pharmacia biotech, Uppsala, Sweden). 31 clones and 23 clones that express the F protein at relatively high levels were obtained from the clones yielded from cells introduced with pCALNdLw-Zeo-F(furin) and pCALNdLw-Zeo-F(5R), respectively.

These clones were tested for the viral productivity of F gene-deficient SeV (SeV/ΔF). First, as a simple and convenient method, cells were infected with SeV/ΔF carrying the GFP gene (SeV/ΔF-GFP) and examined for the spread of GFP fluorescence. Specifically, cells of each clone were plated in 6-well plates and cultured at 37°C. The expression of the F protein was induced by the method described above (AxCANCre infection). The cells were then incubated at 32°C. One day after infection, the cells were infected with SeV/ΔF carrying GFP gene (SeV/ΔF-GFP) at a MOI of 0.1. The spread of GFP fluorescence was monitored under a fluorescence microscope over time. The spread of GFP fluorescence was detected in 8 clones introduced with pCALNdLw-Zeo-F(furin) and 12 clones introduced with pCALNdLw-Zeo-F(5R). These clones were tested for the viral productivity. After infection of SeV/ΔF-GFP at a MOI of 0.5, the cells were cultured at 32°C in the presence or absence of 10% FBS. Three days after infection, virus titers of the culture supernatants were determined. For every clone, the titer was 10 to 20 times higher in the presence of 10% FBS than in the absence of FBS. In particular, when clone F5R2 was used, the productivity was high and 3 x 10⁶ CIU/ml of SeV/ΔF-GFP was harvested in the presence of 10% FBS. It was confirmed that SeV/ΔF could be produced using a HeLa cell, which could not previously be used as a packaging cell. Moreover, SeV/ΔF was successfully produced in the presence of serum without addition of trypsin.

### [Example 10] Improvement of vector productivity

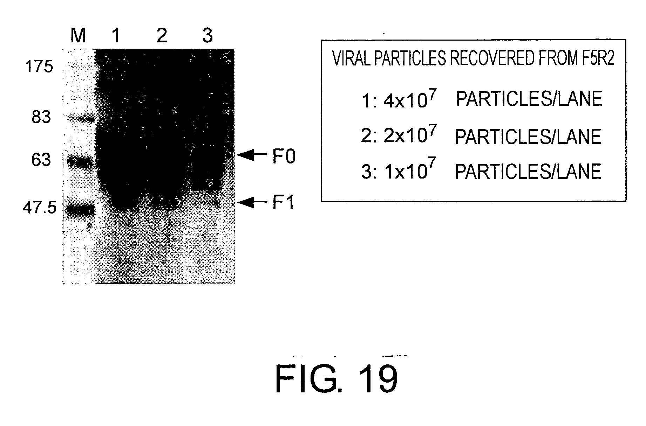

The vector productivity of clone F5R2 was relatively high (3 x 10⁶ CIU/ml). However, the productivity needs to be further improved when the clone is practically used to produce a vector. The SeV vector (SeV/ΔF-GFP) produced with F5R2 was examined for the degree of F protein cleavage by Western-blotting. An anti-F1 antibody was used to achieve this test. The anti-F1 antibody was a newly prepared polyclonal antibody, which was prepared from serum of a rabbit immunized with a mixture of three synthetic peptides covering amino acids 1 to 8 (FFGAVIGT+Cys/SEQ ID NO: 52), amino acids 27 to 39 (EAREAKRDIALIK/SEQ ID NO: 53), and amino acids 285 to 297 (CGTGRRPISQDRS/SEQ ID NO: 54) of F1 protein. Both F0 and F 1 can be detected by the anti-F 1 antibody. The induction of F protein expression and the vector production were carried out by the same method as described in Example 9. Since the anti-F1 antibody used as a primary antibody for Western blotting was a polyclonal antibody, an anti-rabbit IgG antibody (anti-rabbit IgG (Goat) H+L conj.; ICN P., Aurola, OH) was used as a secondary antibody. The result of detecting the F protein in the SeV vector (SeV/ΔF-GFP) produced with F5R2 cells is shown in Fig. 19. The rate of F1, which is an activated F protein, was found to be relatively small.

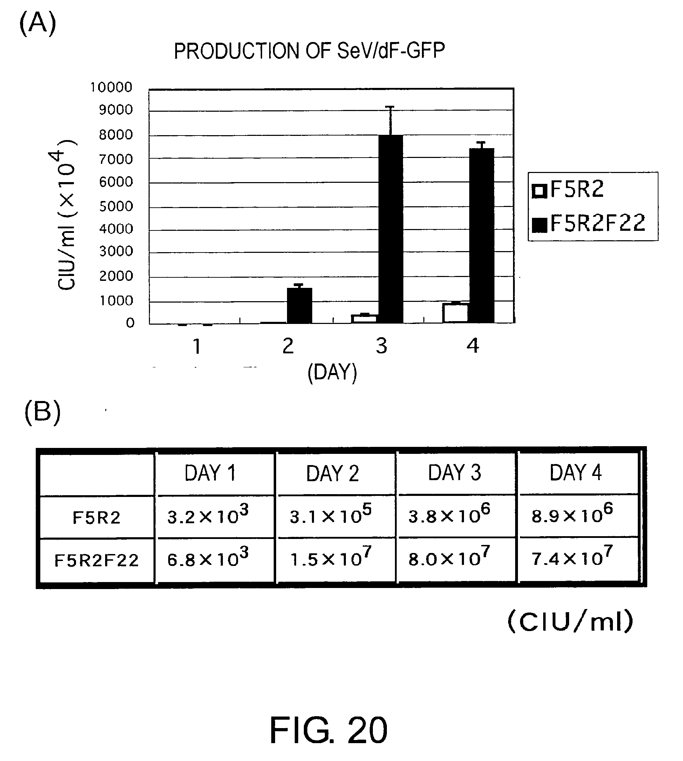

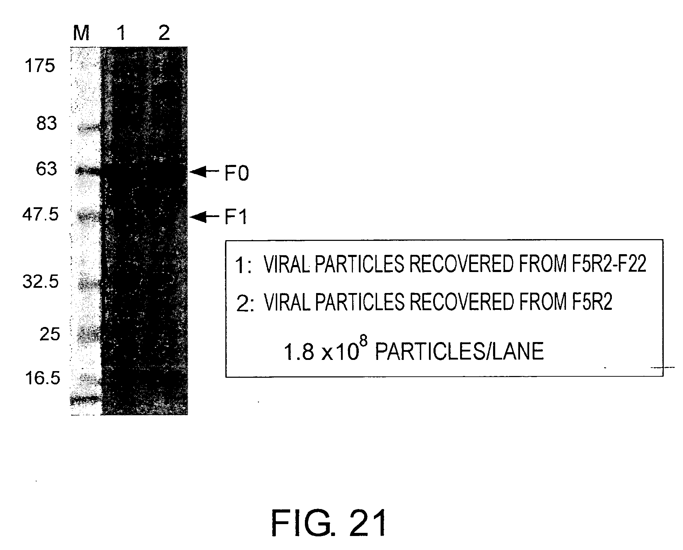

To obtain modified F protein-expressing cells with higher virus productivity, cells with an increased F1 cleavage frequency were selected through re-cloning of FSR2 cells. 32 clones were yielded by the selection, and tested for the vector productivity. As a result, clone F5R2-F22 was found to exhibit the highest productivity. After infection of SeV/ΔF-GFP at a MOI of 0.5, the clone was cultured in the presence of 10% FBS at 32°C. The result of monitoring the titer of SeV/ΔF-GFP in the culture supernatant over time is shown in Fig. 20. Re-cloned F5R2-F22 exhibited more than 10 times higher productivity than F5R2, the parental cell, and was able to produce 8 x 10⁷ CIU/ml of SeV/ΔF-GFP three days after infection. The degree of cleavage of F protein derived from the SeV vector (SeV/ΔF-GFP) produced by the cells (clone F5R2-F22) was also estimated by Western blotting using the anti-F1 antibody. The degree of F1 cleavage was found to be increased compared to F5R2 cells (Fig. 21). This was thought to contribute to the improvement of productivity (= production of infectious viral particles). A possible approach to further improving the productivity is, for example, to increase the furin-like activity by introducing human furin gene or a gene for a protein having a similar activity into cells. Since the produced SeV vector (SeV/ΔF-GFP) contains a relatively large amount of F protein, it is expected that the productivity can be further improved by increasing the ratio of the active form of F protein (F 1 protein) through the acceleration of F0 protein cleavage.

### Industrial Applicability

The methods of the present invention enables the production of viruses without using a protease that is originally required for propagation of the viruses. The use of a cleavage sequence for a protease expressed endogenously in virus-producing cells, or a protease that hardly produces adverse effects on cells, widens the range of selection of cells used to produce viruses, thus improving the efficiency of virus production. The methods of the present invention are useful as methods for producing desired gene transfer vectors including vectors for gene therapy.

## Claims

1. A method for producing a virus whose propagation depends on cleavage of a viral protein by a protease, wherein the method comprises the step of producing the virus in the presence of:
(i) a modified viral protein in which a cleavage sequence for the protease is changed to a cleavage sequence for an alternative protease, and (ii) the alternative protease, and wherein the produced virus comprises the modified viral protein that is cleaved but does not comprise a gene encoding the modified viral protein.

2. The method of claim 1, wherein the produced virus carries a gene encoding the relevant viral protein comprising a wild type cleavage sequence.

3. The method of claim 1, wherein the produced virus is a nontransmissible virus that lacks a gene encoding the relevant viral protein.

4. The method of claim 1, wherein the alternative protease is endogenously expressed in a cell producing the virus.

5. The method of claim 1, wherein the alternative protease is furin.

6. The method of claim 1, wherein the cleavage sequence for the alternative protease comprises Arg-Xaa-Lys/Arg-Arg.

7. The method of claim 1, wherein the cleavage sequence for the alternative protease comprises Arg-Arg-Arg-Arg.

8. The method of claim 1, wherein the virus is a minus-strand RNA virus.

9. The method of claim 8, wherein the minus-strand RNA virus is a Paramyxoviridae virus.

10. The method of claim 8, wherein the minus-strand RNA virus is Sendai virus.

11. A vector which encodes a modified viral protein in which a cleavage sequence for a protease of a viral protein in a virus whose propagation depends on cleavage of the viral protein by the protease is changed to a cleavage sequence for an alternative protease, wherein the vector is a viral or non-viral vector that cannot propagate in a cell producing the virus.

12. The vector of claim 11, which is a plasmid.

13. The vector of claim 11, wherein the expression of the modified viral protein can be induced by a recombinase.

14. The vector of claim 13, wherein the recombinase is Cre or Flp.

15. The vector of claim 11, wherein the alternative protease is expressed endogenously in the cell producing the virus.

16. The vector of claim 11, wherein the alternative protease is furin.

17. The vector of claim 11, wherein the cleavage sequence for the alternative protease comprises Arg-Xaa-Lys/Arg-Arg.

18. The vector of claim 11, wherein the cleavage sequence for the alternative protease comprises Arg-Arg-Arg-Arg.

19. The vector of claim 11, wherein the viral protein is F protein of a minus-strand RNA virus.

20. The vector of claim 19, wherein the minus-strand RNA virus is a Paramyxoviridae virus.

21. The vector of claim 19, wherein the minus-strand RNA virus is Sendai virus.

22. A mammalian cell containing the vector of claim 11.

23. The cell of claim 22, which is a cell for producing a virus whose propagation depends on cleavage of a viral protein by a protease.

24. The cell of claim 22, wherein a gene encoding the modified viral protein is integrated into a chromosome of the cell.

25. The cell of claim 22, which is a human cell.

26. A modified virus of a virus whose propagation depends on cleavage of a viral protein by a protease, wherein the modified virus comprises a modified viral protein in which a cleavage sequence of the viral protein for the protease is changed to a cleavage sequence for an alternative protease, and wherein the modified virus does not comprise a gene encoding the modified viral protein.

27. The modified virus of claim 26, wherein a produced virus carries a gene encoding the relevant viral protein comprising a wild type cleavage sequence.

28. The modified virus of claim 26, which is a nontransmissible virus lacking a gene encoding the relevant viral protein.

29. The modified virus of claim 26, wherein the alternative protease is expressed endogenously in a cell producing the virus.

30. The modified virus of claim 26, wherein the alternative protease is furin.

31. The modified virus of claim 26, wherein the cleavage sequence for the alternative protease comprises Arg-Xaa-Lys/Arg-Arg.

32. The modified virus of claim 26, wherein the cleavage sequence for the alternative protease comprises Arg-Arg-Arg-Arg.

33. The modified virus of claim 26, wherein the virus is a minus-strand RNA virus and the viral protein is F protein.

34. The modified virus of claim 33, wherein the minus-strand RNA virus is a Paramyxoviridae virus.

35. The modified virus of claim 33, wherein the minus-strand RNA virus is Sendai virus.
